# EUROPEAN PATENT APPLICATION

(11) **EP 3 466 263 A1**
(43) Date of publication of application: **10.04.2019**
(21) Application number: 17807038.9
(22) Date of filing: 01.06.2017
(51) Int. Cl.: A01N 25/28, A01N 59/14, A61K 8/02, A61Q 19/00, A61Q 17/00, A61K 9/14, A61K 9/51, A23L 3/358, A23L 29/00

(54) **ANTIMICROBIAL AGENT COMPRISING CARBON-GROUP NON-OXIDE NANOPARTICLES, AND PRODUCTION METHOD THEREFOR**

(30) Priority: 01.06.2016 KR 20160068383; 22.09.2016 KR 20160121436; 28.10.2016 KR 20160141895; 03.11.2016 KR 20160145857; 01.12.2016 KR 20160163088; 21.03.2017 KR 20170035634
(71) Applicant: Shonano Co., Ltd., Jung-gu, Ulsan 44412 (KR)
(72) Inventor: CHO, Won Il, Busan 46566 (KR)
(74) Representative: Botti, Mario
(86) International application number: PCT/KR2017/005748
(87) International publication number: WO 2017/209545

(57) **Abstract**

One embodiment of the present invention provides: an antimicrobial agent comprising carbon-group non-oxide nanoparticles having an average particle size of 5 to 400 nm; and a production method therefor.

## Description

### [Technical Field]

The present invention relates to an antimicrobial agent comprising carbon-group non-oxide nanoparticles, and a production method therefor.

### [Background Art]

The carbon-group (Group IVA or Group XIV) elemental nanoparticles have been of great interest to many researchers as the core elements of applications as next-generation silicon-based optoelectronic devices and developments of nano devices, and carbon-group nanoparticles are widely applied in field effect transistors (TFTs) of displays, solar cells using PN junctions, diodes, display systems of living bodies, and the like.

In the related art, a method for reducing a silicon source such as silicon tetrachloride and silicon triethyl orthosilicate by a chemical method has been used as the most general method for producing silicon nanoparticles, which is one of the carbon group, but this method has problems in that silicon nanoparticles are excessively capped and impurities such as carbon remain after the oxidation and sintering process of silicon nanoparticles.

In order to solve the problems, a method for depositing the silicon source by the vapor-liquid-solid (VLS) mechanism or solid-liquid-solid (SLS) mechanism has been used, but this method also has problems in that since the silicon source is deposited at high pressure and high temperature, not only the reaction conditions are severe but also handling is difficult and expensive equipment is necessary, and the yield of silicon nanoparticles is low.

Meanwhile, in the related art, carbon-group nanoparticles such as silicon have been usually applied to electronic products and the like, and there has been no example in which the surface of the carbon group nanoparticles is modified and used as an antimicrobial agent.

### <Prior Art Documents>

### <Patent Documents>

(Patent Document 0001) Korean Patent Application Laid-Open No. 10-2012-0010901 (February 6, 2012)
(Patent Document 0002) Korean Patent Application Laid-Open No. 10-2014-0072663 (June 13, 2014)

### [Disclosure]

### [Technical Problem]

The present invention has been made in an effort to solve the above-described problems in the related art, and an object of the present invention is to provide an antimicrobial agent exhibiting excellent antimicrobial effects even in a small amount thereof and comprising carbon-group non-oxide nanoparticles of which the production costs are inexpensive due to the simple production process thereof, and a production method therefor.

### [Technical Solution]

An aspect of the present invention provides an antimicrobial agent comprising carbon-group non-oxide nanoparticles having an average particle size of 5 to 400 nm.

The present inventors have found that the carbon-group non-oxide nanoparticles of the present invention exhibit remarkably excellent antimicrobial effects comparable to those of organic antimicrobial agents in the related art, thereby completing the present invention.

The term "carbon-group non-oxide nanoparticles as used herein refers to particles including at least one carbon-group (Group XIV) element of C, Si, Ge, and Sn, and further including B, if necessary, and may be understood as a concept including particles of an alloy of heterogeneous carbon-group elements or an alloy in which boron (B) is alloyed with at least one carbon-group element, a compound consisting of heterogeneous carbon-group elements, and a compound consisting of a carbon-group element and boron.

For example, the carbon-group non-oxide nanoparticles may be Si nanoparticles, Si/B alloy nanoparticles, SiBₓ nanoparticles, Si/C alloy nanoparticles, Si/Ge alloy nanoparticles, Si/Ge/B alloy nanoparticles, Si/Ge/C alloy nanoparticles, Ge nanoparticles, Ge/B alloy nanoparticles, GeBₓ nanoparticles, Ge/C alloy nanoparticles, C/B alloy nanoparticles, CB₄ nanoparticles, and Sn nanoparticles.

The term "non-oxide nanoparticles" as used herein refers to particles which do not substantially include an oxygen element (O), and may be understood as a concept including an oxide layer (a first oxide layer) produced on the surface of non-oxide nanoparticles by a naturally occurring oxidation reaction.

The first oxide layer may have a thickness of preferably 1 nm. Since carbon-group non-oxide nanoparticles having the first oxide layer formed in a thickness of 1 nm or less have are highly reactive with alcohols, carboxylic acids, water, and the like, the efficiency in which the surface is modified with an alkoxy group, a carboxyl group, and a hydroxyl group is improved and the dispersibility due to electric repulsive force among particles is excellent.

The antimicrobial effect of the carbon-group non-oxide nanoparticles may be realized by the following mechanism. First, an antigenic material is present on the surface of bacteria, and when carbon-group non-oxide nanoparticles such as silicon nanoparticles come into contact with and are adsorbed onto these bacteria, the antigenic material acts as an agglutinogen to stimulate the production of an agglutinin, which is an antibody thereof, thereby leading to the formation of an aggregate. The carbon-group non-oxide nanoparticles adsorbed on the surface of bacteria may penetrate into the bacteria by the movement (contraction, expansion) of the bacterial phospholipids, and as a result, apoptosis of the bacteria may occur. Further, the carbon-group non-oxide nanoparticles may serve as nutrients for the survival of the bacteria. Bacteria attempt to absorb carbon-group non-oxide nanoparticles in order to ingest inorganic nutrients while phospholipids clustered in bacteria are contracting and expanding, but the nanoparticles are caught between the phospholipids due to the size of the nanoparticles. In that case, as the gap between phospholipids is slowly increased, electrolytes and the like inside the bacteria are discharged to the outside, and as a result, apoptosis of the bacteria may occur.

The carbon-group non-oxide nanoparticles may have an average particle size of 5 to 400 nm. When the carbon-group non-oxide nanoparticles have an average particle size of less than 5 nm, an arbitrary particle aggregation may occur when nanoparticles are produced, and when the carbon-group non-oxide nanoparticles have an average particle size of more than 400 nm, an apoptotic effect caused by the mechanism cannot be realized.

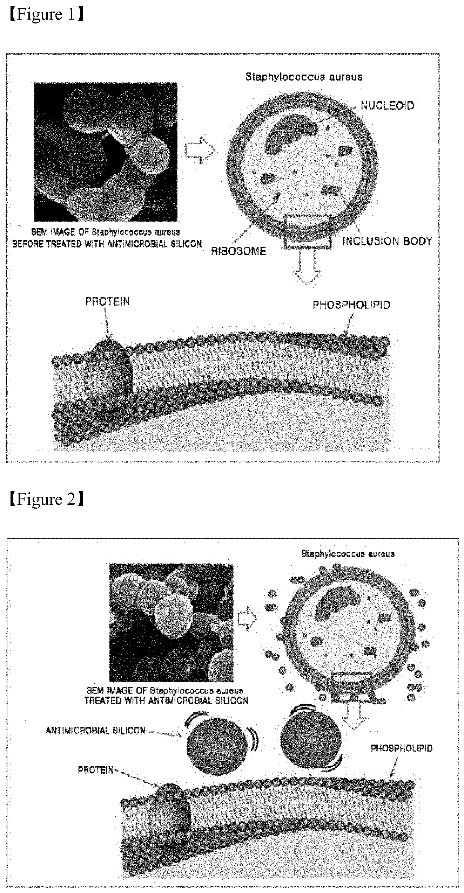

FIGS. 1 to 4 are SEM images before and immediately after an antimicrobial agent consisting of the silicon non-oxide nanoparticles according to one embodiment of the present invention is brought into contact with 50 ppm of *Staphylococcus aureus* and 24 hours after the antimicrobial treatment.

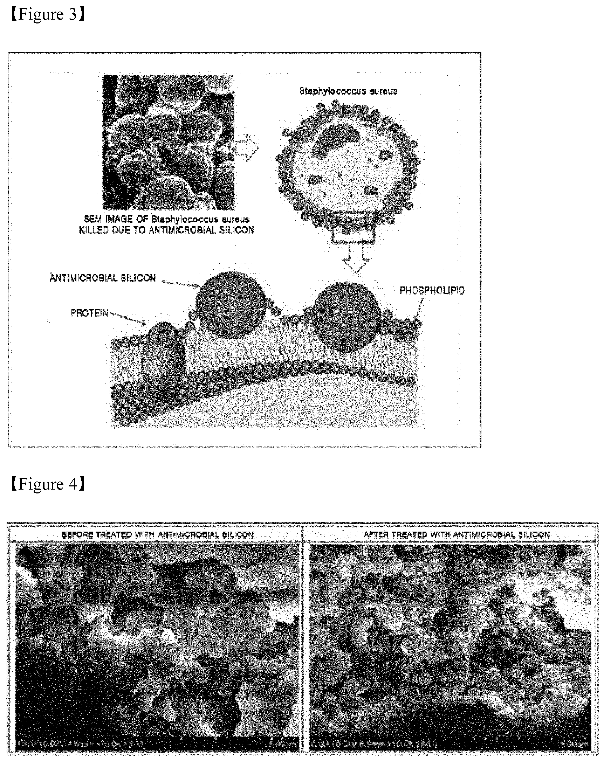

In FIG. 2, it can be seen that silicon non-oxide nanoparticles are brought into contact with and adsorbed onto bacteria immediately after *Staphylococcus aureus* is subjected to antimicrobial treatment, and in FIG. 3, it can be confirmed that the *Staphylococcus aureus* is killed after the antimicrobial treatment. Further, when the results before and 24 hours after the antimicrobial treatment are compared through FIG. 4, *Staphylococcus aureus* had a spherical shape of 500 nm, but after the antimicrobial treatment, the spherical shape is distorted while silicon non-oxide nanoparticles come into contact with and are adsorbed onto the bacteria, and the bacteria are killed.

In addition, the carbon-group non-oxide nanoparticles may be nanoparticles having a carbon layer having a certain thickness, for example, 100 nm or less, preferably 1 to 100 nm, and more preferably 1 to 10 nm formed on the surface of the particle. The carbon layer allows the antimicrobial performance of carbon-group non-oxide nanoparticles to be stably realized by preventing peroxidation caused by contact of carbon-group non-oxide nanoparticles with the air and the accompanying thickening of the first oxide layer. When the carbon layer has a thickness of less than 1 nm, it is difficult to appropriately block contact of the nanoparticles with the air, and when the carbon layer has a thickness of more than 100 nm, the nanoparticles are excessively enlarged, so that the apoptotic effect by the mechanism cannot be realized.

Meanwhile, the carbon-group non-oxide nanoparticles may further include one or more functional groups selected from the group consisting of a carboxyl group, a hydroxyl group, and an alkoxy group, which are bonded to the surface of the first oxide layer.

The carboxyl group, the hydroxyl group, and the alkoxy group may be bonded to the surface of the carbon-group non-oxide nanoparticles, specifically, the surface of the first oxide layer by being subjected to treatment with an alcohol, a carboxylic acid, an aqueous boron solution, water, and the like.

Since the carbon-group non-oxide nanoparticles having the functional group bonded to the surface thereof exhibit excellent antimicrobial effects, have a simplified production method thereof, and exhibit excellent antimicrobial effects even when the nanoparticles are used in a small amount, the carbon-group non-oxide nanoparticles have excellent coating power with respect to various products such as various electronic products, clothes, bags, and shoes, so that versatility and stability are excellent.

The carbon-group non-oxide nanoparticles may further include a second oxide layer consisting of a boron oxide formed on the surface of the first oxide layer. The second oxide layer allows the antimicrobial performance of carbon-group non-oxide nanoparticles to be stably realized by preventing peroxidation caused by contact of carbon-group non-oxide nanoparticles with the air and the accompanying thickening of the first oxide layer.

If necessary, the antimicrobial agent may be provided in the form of a solution diluted to a certain concentration, for example, a concentration of 1 to 1,000 ppm, by water, and the like, but the type of medium with which the antimicrobial agent may be diluted is not particularly limited.

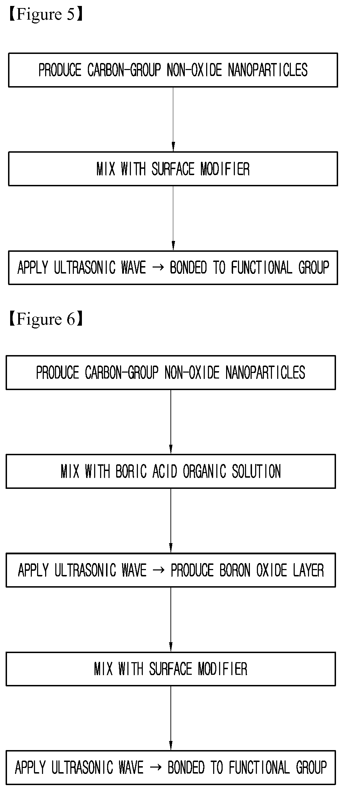

Referring to FIG. 5, another aspect of the present invention provides a method for producing an antimicrobial agent, the method including the steps of: (a) producing carbon-group non-oxide nanoparticles having a first oxide layer formed thereon; (b1) producing a mixed solution by mixing the carbon-group non-oxide nanoparticles with one selected from the group consisting of an alcohol, a carboxylic acid, an aqueous boric acid solution, and water; and (c1) applying ultrasonic waves to the mixed solution.

Referring to FIG. 6, still another aspect of the present invention provides a method for producing an antimicrobial agent, the method including the steps of: (a) producing carbon-group non-oxide nanoparticles having a first oxide layer formed thereon; (b2) producing a first mixed solution by mixing the carbon-group non-oxide nanoparticles with a first solution including boric acid and an organic solvent; (c2) obtaining carbon-group non-oxide nanoparticles having a second oxide layer consisting of a boron oxide formed on the first oxide layer by applying ultrasonic waves to the first mixed solution; (d) producing a second mixed solution by mixing the carbon-group non-oxide nanoparticles with one selected from the group consisting of an alcohol, a carboxylic acid, an aqueous boric acid solution, and water; and (e) applying ultrasonic waves to the second mixed solution.

Step (a) may be performed by irradiating a mixed gas including one or more raw material gases including a carbon-group element and a sulfur hexafluoride catalyst gas with laser.

The alcohol and the carboxylic acid usable in Step (b) may be one or more selected from the group consisting of 1,10-decanediol, 1,2-propanediol, 1,2-hexanediol, 1,4-butanediol, 1,5-pentanediol, 1,8-octanediol, 1-decanol, 2,2,4-trimethyl pentane diol, 2-butoxyethanol, 2-bromopentanoic acid, 2-bromohexadecanoic acid, 2-bromohexanoic acid, 2-ethylhexanoic acid, 2-chlorobutanoic acid, 2-propanediol, 2-propene acid, 2-hydroxyethyl methacrylate, DHA, galatamine, galactose, galantamine, citric acid, glycine, gluconate, glucose, glutaric acid, glutamine, glycerol, glycyrrhetinic acid, dipotassium glycyrrhizinate, glycine, sodium gluconate, sodium carboxymethyl cellulose, neomycin sulfate, neopentyl glycol, doxorubicin, diglyceride, diethylene glycol, dipropylene glycol, lanolin, lactic acid, retinol, linoleic acid, maleic acid, methacrylic acid, methanol, methoxypropanol, metformin, methylene chloride, menthol, monoglyceride, mineral oil, vaseline, beta-phenyl ethyl alcohol, benzoic acid, benzyl alcohol, butanol, butyl phenol, butyric acid, butyl hydroxyanisole (BHA), bromelain, bisphenol A, vitamin C, vitamin D, ciclopirox, physiological saline, serrapeptase, cetearyl alcohol, cetyl alcohol, cellulose, sodium citrate, sorbitol, sofalcone, stearic acid, stearyl alcohol, adipic acid, adipinic acid, aryl alcohol, aminolevulinic acid, amino alcohol, aceclofenac, aceclofenac acid, acetic acid, acetaminophen, acetylsalicylic acid, azolene, alginic acid, illite, alendronic acid, ergosterol, ethanol, ethylene glycol, ethylene vinyl alcohol, octanol, eugenol, ibuprofen, isopentyldiol, isopropyl alcohol, isophthalic acid, itaconic acid, xylitol, carbazochrome, casa triol, crotyl alcohol, chloroxylenol, clobetasol propionate, tannic acid, terephthalic acid, tranexamic acid, triamcinolone acetonide, timolol, palmitoleic acid, phenoxyethanol, pectin, pentaerythritol, a polyacrylic acid ammonium salt, a polyester polyol, polyethylene glycol, fusidic acid, prednisolone, propanol, propolis, propionic acid, propylene glycol, propionic acid, fibroin, heparin, hexanedioic acid, flubendazole, hinokitiol, and hyaluronic acid, but are not limited thereto.

The method may further comprise the step of forming a carbon layer on the surface of the carbon-group non-oxide nanoparticles by irradiating a mixture including the carbon-group non-oxide nanoparticles and a carbon-based gas, for example, an acetylene gas or an ethylene gas with laser after Step (a).

In Step (b2), the organic solvent may be non-polar. When the organic solvent is non-polar, a second oxide layer consisting of a boron oxide may be formed on the first oxide layer in Step (c2) according to the following Reaction Formula A. In contrast, when a boron solution dissolved in a polar solvent such as water is treated in Step (b2), an additional oxide layer consisting of a boron oxide is not produced, but a boron-derived hydroxyl group is bonded to the surface of the first oxide layer.

<Reaction Formula A> Nanoparticles + B(OH)₃ → Nanoparticles-OB(OH)₂ + 1/2 H₂ → Nanoparticles(-O)₂BOH + 2/2 H₂ → Nanoparticles(-O)₃B + 3/2 H₂

In Step (c1) or (c2), a functional group may be bonded to the surface of the carbon-group non-oxide nanoparticles by applying ultrasonic waves for a certain time, for example, 4 to 6 minutes.

The process in which the surface of the carbon-group non-oxide nanoparticles is modified into an alkoxy group by the ultrasonic waves is the same as in the following Reaction Formula B.

<Reaction Formula B> Nanoparticles + nROH → Nanoparticles-(O-R)n + n/2H₂↑

(R is an alkyl group or an alkylketone group, an aromatic group, or an aromatic ketone group)

When the time for irradiating the ultrasonic waves is less than 4 minutes, the bonding efficiency of the functional group with respect to the surface of the carbon-group non-oxide nanoparticles may be reduced, and when the time for irradiating the ultrasonic waves is more than 6 minutes, the energy efficiency may be reduced because more ultrasonic waves than needed are applied.

The frequency of the ultrasonic wave is not particularly limited, and any frequency can be used as long as the frequency is a frequency of an ultrasonic wave typically used, but it is preferred to use an ultrasonic wave within a frequency range of 20 to 100 kHz.

### [Advantageous Effects]

The antimicrobial agent according to an aspect of the present invention and a production method therefor provide an antimicrobial composition exhibiting excellent antimicrobial effects and exhibit excellent effects of decreasing the production costs thereof and simplifying the production processes thereof because carbon-group non-oxide nanoparticles having an oxidized layer formed in a small thickness are used, and therefore, a dispersant or a separate additive need not be used.

Furthermore, since an antimicrobial agent containing carbon-group non-oxide nanoparticles substituted with a functional group exhibiting excellent antimicrobial effects even in a small amount has a low surface tension and excellent coating power, the antimicrobial agent may be applied to various products such as various electronic products, clothes, bags, and shoes, and may also be used in the development of a new drug, or resins, cosmetics, and the like.

The effects of the present invention are not limited to the aforementioned effects, and they should be understood to include all possible effects deduced from the configuration of the invention described in the detailed description or the claims of the present invention.

### [Description of Drawings]

FIG. 1 is an SEM image before the antimicrobial agent according to an embodiment of the present invention is brought into contact with *Staphylococcus aureus.*

FIG. 2 is an SEM image immediately after the antimicrobial agent according to an embodiment of the present invention is brought into contact with *Staphylococcus aureus.*

FIG. 3 is an SEM image 24 hours after the antimicrobial agent according to an embodiment of the present invention is brought into contact with *Staphylococcus aureus.*

FIG. 4 is a set of SEM images for comparison of before the antimicrobial agent according to an embodiment of the present invention is brought into contact with *Staphylococcus aureus* and 24 hours after the antimicrobial agent according to an embodiment of the present invention is brought into contact with *Staphylococcus aureus.*

FIG. 5 illustrates a method for producing the antimicrobial agent according to an embodiment of the present invention.

FIG. 6 illustrates a method for producing the antimicrobial agent according to another embodiment of the present invention.

FIG. 7 is a TEM image of the silicon nanoparticle according to an embodiment of the present invention.

### [Modes of the Invention]

Hereinafter, the present invention will be described with reference to the accompanying drawings. However, the present invention can be realized in various different forms, and is not limited to the Examples described herein. Moreover, in the accompanying drawings, parts that are not related to the description will be omitted in order to clearly describe the present invention.

Throughout the specification, when one part is "connected" to another part, this includes not only a case where they are "directly connected to each other", but also a case where they are "indirectly connected to each other" with another member therebetween. Further, when one part "includes" one constituent element, unless otherwise specifically described, this does not mean that another constituent element is excluded, but means that another constituent element may be further included.

Hereinafter, embodiments of the present invention will be described in detail.

### Preparation Example 1

Silicon nanoparticles can be produced according to (1) or (2) in the following Reaction Formula 1.

<Reaction Formula 1> (1) SiH₄ + SF₆ + N₂ → Si (-S, -F) + H₂ + N₂ (2) SiH₄ + N₂ → Si + 2H₂ + N₂

Silicon nanoparticles (Si-NPs) having an oxidized layer formed thereon were produced by supplying a mixed gas obtained by mixing 100 parts by volume of monosilane (SiH₄) as a raw material gas, 400 parts by volume of nitrogen (N₂) as a control gas, and 40 parts by volume of a sulfur hexafluoride (SF₆) catalyst gas to a reaction chamber having an internal pressure of 500 torr through a raw material gas supply nozzle and irradiating the mixed gas supplied to the reaction chamber with laser generated by a CO₂ laser generator in the form of a line beam of continuous waves having a wavelength of 10.6 µm through a laser irradiation part for 3 hours.

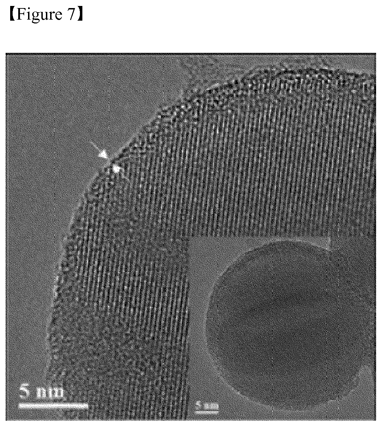

Referring to FIG. 7, the produced silicon nanoparticles having an oxidized layer formed thereon have an average particle size of 5 to 400 nm, the oxidized layer has a thickness of 0.32 nm, and the production yield thereof is 97.1%.

### Preparation Example 2

Silicon-boron alloy nanoparticles or silicon boride nanoparticles can be produced according to the following Reaction Formula 2.

<Reaction Formula 2> 2SiH₄ + 2B₂H₆ + N₂ → SiB₄ + 8H₂ + N₂

Monosilane (SiH₄), diborane (B₂H₆), and nitrogen were mixed, and the resulting mixture was injected into a reaction chamber and irradiated with a CO₂ laser beam. In this case, diborane serves as a catalyst gas and a raw material gas, and produces silicon-boron alloy nanoparticles (SiBx-NPs) by allowing energy absorbed at a wavelength of 10.6 µm to be efficiently transferred to monosilane and allowing a Si-H bond of monosilane to be well broken down.

In addition, diborane is broken down into boron and hydrogen atoms, so that boron forms an alloy with silicon nanoparticles, and prevents oxidation of silicon. Monosilane as a raw material gas is 90% or more of the total volume (a combined volume of the raw material gas and a catalyst gas), and the catalyst gas is adjusted within a range of 10% or less of the total volume. Furthermore, nitrogen as a carrier gas is adjusted so as not to exceed 400 parts by volume with respect to monosilane as a raw material gas. The flow rate of the gas is in units of sccm. The nanoparticles are produced by setting the process pressure inside the reaction chamber within a range of 100 to 400 torr. Within this range, silicon-boron alloy nanoparticles (SiBx-NPs) having an average particle size of 5 to 400 nm and an oxidized layer thickness of 0.57 nm are produced.

### Preparation Example 3

Silicon-carbon alloy nanoparticles or silicon carbide nanoparticles can be produced according to (1) or (2) in the following Reaction Formula 3.

<Reaction Formula 3> (1) 2SiH₄ + C₂H₂ + SF₆ → S + 2SiC + 6HF + 2H₂ (2) 2SiH₄ + C₂H₂ → 2SiC + 5H₂

Monosilane (SiH₄), acetylene (C₂H₂), and nitrogen were mixed, and the resulting mixture was injected into a reaction chamber and irradiated with a CO₂ laser beam. Acetylene is broken down into carbon and hydrogen atoms, so that carbon forms an alloy with silicon nanoparticles, and prevents oxidation of silicon. Monosilane and acetylene as raw material gases are injected at a volume ratio of 2 : 1, respectively. Further, nitrogen as a carrier gas is adjusted so as not to exceed 400 parts by volume with respect to monosilane as a raw material gas. The flow rate of the gas is in units of sccm. The nanoparticles are produced by setting the process pressure inside the reaction chamber within a range of 100 to 500 torr. Within this range, silicon-carbon alloy nanoparticles (SiC-NPs) having an average particle size of 5 to 400 nm and an oxidized layer thickness of 0.53 nm are produced.

### Preparation Example 4

Silicon-germanium alloy nanoparticles can be produced according to (1) or (2) in the following Reaction Formula 4.

<Reaction Formula 4> (1) SiH₄ + GeH₄ + SF₆ → S + SiGe + 6HF + H₂ (2) SiH₄ + GeH₄ → SiGe + 4H₂

Silicon-germanium alloy nanoparticles (SiGe-NPs) were produced by supplying a mixed gas obtained by mixing 100 parts by volume (Raw Material Gas 1) of germane (GeH₄) and 100 parts by volume (Raw Material Gas 2) of monosilane (SiH₄) as raw material gases and 400 parts by volume of nitrogen (N₂) as a carrier gas to a reaction chamber having an internal pressure of 100 to 500 torr through a raw material gas supply nozzle and irradiating the mixed gas supplied to the reaction chamber with a laser generated by a CO₂ laser generator in the form of a line beam of continuous waves having a wavelength of 10.6 µm through a laser irradiation part for 3 hours. The average particle size of the SiGe-NPs was 5 to 400 nm, and the thickness of an oxidized layer formed on the surface thereof was 0.47 nm.

### Preparation Example 5

Silicon-germanium-boron alloy nanoparticles can be produced according to the following Reaction Formula 5.

<Reaction Formula 5> 2SiH₄ + 2GeH₄ + B₂H₆ → 2SiGeB + 11H₂

Silicon-germanium-boron alloy nanoparticles (SiGeB-NPs) were produced by supplying a mixed gas obtained by mixing 100 parts by volume of monosilane (SiH₄), 100 parts by volume of germane (GeH₄), and 40 to 80 parts by volume of diborane (B₂H₆) as raw material gases and 400 parts by volume of nitrogen (N₂) as a carrier gas to a reaction chamber having an internal pressure of 80 to 400 torr through a raw material gas supply nozzle and irradiating the mixed gas supplied to the reaction chamber with a laser generated by a CO₂ laser generator in the form of a line beam of continuous waves having a wavelength of 10.6 µm through a laser irradiation part for 3 hours. The average particle size of the SiGeB-NPs was 5 to 400 nm, and the thickness of an oxidized layer formed on the surface thereof was 0.75 nm.

### Preparation Example 6

Silicon-germanium-carbon alloy nanoparticles can be produced according to the following Reaction Formula 6.

<Reaction Formula 6> 2SiH₄ + 2GeH₄ + C₂H₂ → 2SiGeC + 9H₂

Silicon-germanium-carbon alloy nanoparticles (SiGeC-NPs) were produced by supplying a mixed gas obtained by mixing 100 parts by volume of monosilane (SiH₄), 100 parts by volume of germane (GeH₄), and 40 to 80 parts by volume of acetylene (C₂H₂) as raw material gases and 400 parts by volume of nitrogen (N₂) as a carrier gas to a reaction chamber having an internal pressure of 80 to 400 torr through a raw material gas supply nozzle and irradiating the mixed gas supplied to the reaction chamber with a laser generated by a CO₂ laser generator in the form of a line beam of continuous waves having a wavelength of 10.6 µm through a laser irradiation part for 3 hours. The average particle size of the SiGeC-NPs was 5 to 400 nm, and the thickness of an oxidized layer formed on the surface thereof was 0.68 nm.

### Preparation Example 7

Germanium nanoparticles can be produced according to (1) or (2) in the following Reaction Formula 7.

<Reaction Formula 7> (1) 2GeH4 + SF6 → S + 2GeF4 + 2HF + 3H₂ (2) GeH₄ + N₂ → 2Ge + 2H₂ + N₂

Germanium nanoparticles (Ge-NPs) having an oxidized layer formed were produced by supplying a mixed gas obtained by mixing 100 parts by volume of germane (GeH₄), 400 parts by volume of hydrogen (H₂) as a control gas, and 40 parts by volume of a sulfur hexafluoride (SF₆) catalyst gas to a reaction chamber having an internal pressure of 500 torr through a raw material gas supply nozzle and irradiating the mixed gas supplied to the reaction chamber with a laser generated by a CO₂ laser generator in the form of a line beam of continuous waves having a wavelength of 10.6 µm through a laser irradiation part for 3 hours.

The produced germanium nanoparticles having an oxidized layer formed thereon have an average particle size of 5 to 400 nm, the oxidized layer has a thickness of 0.47 nm, and the production yield thereof is 98.7%.

### Preparation Example 8

Germanium-boron alloy nanoparticles or germanium boride nanoparticles can be produced according to the following Reaction Formula 8.

<Reaction Formula 8> 2GeH₄ + 2B₂H₆ + N₂ → GeB₄ + 8H₂ + N₂

Germanium-boron alloy nanoparticles (GeBx-NPs) were produced by supplying a mixed gas obtained by mixing 100 parts by volume of germane (GeH₄) and 40 to 80 parts by volume of diborane (B₂H₆) as raw material gases and 400 parts by volume of nitrogen (N₂) as a carrier gas to a reaction chamber having an internal pressure of 100 to 400 torr through a raw material gas supply nozzle and irradiating the mixed gas supplied to the reaction chamber with a laser generated by a CO₂ laser generator in the form of a line beam of continuous waves having a wavelength of 10.6 µm through a laser irradiation part for 3 hours. The particle size of the GeBx-NPs was 5 to 400 nm, and the thickness of an oxidized layer formed on the surface thereof was 0.52 nm.

### Preparation Example 9

Germanium-carbon alloy nanoparticles or germanium carbide nanoparticles can be produced according to (1) or (2) in the following Reaction Formula 9.

<Reaction Formula 9> (1) 2GeH₄ + C₂H₂ + SF₆ → S + 2GeC + 6HF + 2H₂ (2) 2GeH₄ + C₂H₂ → 2GeC + 5H₂

Germane (GeH₄), acetylene (C₂H₂), and nitrogen were mixed, and the resulting mixture was injected into a reaction chamber and irradiated with a CO₂ laser beam. Acetylene is broken down into carbon and hydrogen atoms, so that carbon forms an alloy with silicon nanoparticles, and prevents oxidation of silicon. Germane and acetylene as raw material gases are injected at a volume ratio of 2 : 1, respectively. Furthermore, nitrogen as a carrier gas is adjusted so as not to exceed 400 parts by volume with respect to a silane gas as a raw material gas. The flow rate of the gas is in units of sccm. Nanoparticles are produced by setting the process pressure inside the reaction chamber within a range of 100 to 500 torr. Within this range, germanium-carbon alloy nanoparticles (GeC-NPs) having an average particle size of 5 to 400 nm and an oxidized layer thickness of 0.58 nm are produced.

### Preparation Example 10

Carbon-boron alloy nanoparticles or boron carbide nanoparticles can be produced according to the following Reaction Formula 10.

<Reaction Formula 10> C₂H₂ + 4B₂H₆ + N₂ → 2B₄C + 13H₂ + N₂

Carbon-boron alloy nanoparticles (CBx-NPs) were produced by supplying a mixed gas obtained by mixing 100 parts by volume of acetylene (C₂H₂) and 400 parts by volume of diborane (B₂H₆) as raw material gases and 400 parts by volume of nitrogen (N₂) as a carrier gas to a reaction chamber having an internal pressure of 100 to 400 torr through a raw material gas supply nozzle and irradiating the mixed gas supplied to the reaction chamber with a laser generated by a CO₂ laser generator in the form of a line beam of continuous waves having a wavelength of 10.6 µm through a laser irradiation part for 3 hours. The particle size of the CBx-NPs was 5 to 400 nm, and the thickness of an oxidized layer formed on the surface thereof was 0.46 nm.

### Preparation Example 11

An acetylene gas (C₂H₂) and nitrogen were mixed, the resulting mixture was injected into a reaction chamber, Si-NPs having a particle size of 5 to 400 nm in Preparation Example 1 were flowed into the reaction chamber, and then the resulting mixture was irradiated with a CO₂ laser beam. In this case, a C-H bond of the acetylene gas may produce a carbon layer on the surface of the Si-NPs. The nitrogen gas prevents oxidation of Si-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the Si-NPs are coated with carbon (C@Si-NPs), an inner core is a Si-NP having a size of 5 to 400 nm, and a carbon layer within a range of 1 to 100 nm is formed on the surface thereof.

### Preparation Example 12

An acetylene gas (C₂H₂) and nitrogen were mixed, the resulting mixture was injected into a reaction chamber, SiB₄-NPs having a particle size of 5 to 400 nm in Preparation Example 2 were flowed into the reaction chamber, and then the resulting mixture was irradiated with a CO₂ laser beam. In this case, a C-H bond of the acetylene gas may produce a carbon layer on the surface of the SiB₄-NPs. The nitrogen gas prevents oxidation of SiB₄-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the SiB₄-NPs are coated with carbon (C@SiB₄-NPs), an inner core is a SiB₄-NP having a size of 5 to 400 nm, and a carbon layer within a range of 1 to 100 nm is formed on the surface thereof.

### Preparation Example 13

An acetylene gas (C₂H₂) and nitrogen were mixed, the resulting mixture was injected into a reaction chamber, SiC-NPs having a particle size of 5 to 400 nm in Preparation Example 3 were flowed into the reaction chamber, and then the resulting mixture was irradiated with a CO₂ laser beam. In this case, a C-H bond of the acetylene gas may produce a carbon layer on the surface of the SiC-NPs. The nitrogen gas prevents oxidation of SiC-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the SiC-NPs are coated with carbon (C@SiC-NPs), an inner core is a SiC-NP having a size of 5 to 400 nm, and a carbon layer within a range of 1 to 100 nm is formed on the surface thereof.

### Preparation Example 14

An acetylene gas (C₂H₂) and nitrogen were mixed, the resulting mixture was injected into a reaction chamber, SiGe-NPs having a particle size of 5 to 400 nm in Preparation Example 4 were flowed into the reaction chamber, and then the resulting mixture was irradiated with a CO₂ laser beam. In this case, a C-H bond of the acetylene gas may produce a carbon layer on the surface of the SiGe-NPs. The nitrogen gas prevents oxidation of SiGe-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the SiGe-NPs are coated with carbon (C@SiGe-NPs), an inner core is a SiGe-NP having a size of 5 to 400 nm, and a carbon layer within a range of 1 to 100 nm is formed on the surface thereof.

### Preparation Example 15

An acetylene gas (C₂H₂) and nitrogen were mixed, the resulting mixture was injected into a reaction chamber, SiGeB-NPs having a particle size of 5 to 400 nm in Preparation Example 5 were flowed into the reaction chamber, and then the resulting mixture was irradiated with a CO₂ laser beam. In this case, a C-H bond of the acetylene gas may produce a carbon layer on the surface of the SiGeB-NPs. The nitrogen gas prevents oxidation of SiGeB-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the SiGeB-NPs are coated with carbon (C@SiGeB-NPs), an inner core is a SiGeB-NP having a size of 5 to 400 nm, and a carbon layer within a range of 1 to 100 nm is formed on the surface thereof.

### Preparation Example 16

An acetylene gas (C₂H₂) and nitrogen were mixed, the resulting mixture was injected into a reaction chamber, SiGeC-NPs having a particle size of 5 to 400 nm in Preparation Example 6 were flowed into the reaction chamber, and then the resulting mixture was irradiated with a CO₂ laser beam. In this case, a C-H bond of the acetylene gas may produce a carbon layer on the surface of the SiGeC-NPs. The nitrogen gas prevents oxidation of SiGeC-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the SiGeC-NPs are coated with carbon (C@SiGeC-NPs), an inner core is a SiGeC-NP having a size of 5 to 400 nm, and a carbon layer within a range of 1 to 100 nm is formed on the surface thereof.

### Preparation Example 17

An acetylene gas (C₂H₂) and nitrogen were mixed, the resulting mixture was injected into a reaction chamber, Ge-NPs having a particle size of 5 to 400 nm in Preparation Example 7 were flowed into the reaction chamber, and then the resulting mixture was irradiated with a CO₂ laser beam. In this case, a C-H bond of the acetylene gas may produce a carbon layer on the surface of the Ge-NPs. The nitrogen gas prevents oxidation of Ge-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the Ge-NPs are coated with carbon (C@Ge-NPs), an inner core is a Ge-NP having a size of 5 to 400 nm, and a carbon layer within a range of 1 to 100 nm is formed on the surface thereof.

### Preparation Example 18

An acetylene gas (C₂H₂) and nitrogen were mixed, the resulting mixture was injected into a reaction chamber, GeB₄-NPs having a particle size of 5 to 400 nm in Preparation Example 8 were flowed into the reaction chamber, and then the resulting mixture was irradiated with a CO₂ laser beam. In this case, a C-H bond of the acetylene gas may produce a carbon layer on the surface of the GeB₄-NPs. The nitrogen gas prevents oxidation of GeB₄-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the GeB₄-NPs are coated with carbon (C@B₄-NPs), an inner core is a GeB₄-NP having a size of 5 to 400 nm, and a carbon layer within a range of 1 to 100 nm is formed on the surface thereof.

### Preparation Example 19

An acetylene gas (C₂H₂) and nitrogen were mixed, the resulting mixture was injected into a reaction chamber, GeC-NPs having a particle size of 5 to 400 nm in Preparation Example 9 were flowed into the reaction chamber, and then the resulting mixture was irradiated with a CO₂ laser beam. In this case, a C-H bond of the acetylene gas may produce a carbon layer on the surface of the GeC-NPs. The nitrogen gas prevents oxidation of GeC-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the GeC-NPs are coated with carbon (C@GeC-NPs), an inner core is a GeC-NP having a size of 5 to 400 nm, and a carbon layer within a range of 1 to 100 nm is formed on the surface thereof.

### Preparation Example 20

An acetylene gas (C₂H₂) and nitrogen were mixed, the resulting mixture was injected into a reaction chamber, CB₄-NPs having a particle size of 5 to 400 nm in Preparation Example 10 were flowed into the reaction chamber, and then the resulting mixture was irradiated with a CO₂ laser beam. In this case, a C-H bond of the acetylene gas may produce a carbon layer on the surface of the CB₄-NPs. The nitrogen gas prevents oxidation of CB₄-NPs.

An acetylene gas as a raw material gas is contained at 60 or more of the total parts by volume (a combined part by volume of the acetylene gas and the nitrogen gas), and the nitrogen gas is adjusted within a range not exceeding 40 or more of the total parts by volume. The flow rate of the gas is in units of sccm. Nanoparticles are produced by setting the process pressure inside the reaction chamber within a range of 100 to 400 torr. Within this range, when the CB₄-NPs are coated with carbon (C*@*CB₄-NPs), an inner core is a CB₄-NP having a size of 5 to 400 nm, and a carbon layer within a range of 1 to 100 nm is formed on the surface thereof.

### Example 1

Si-NPs surface-modified with a methoxy group were produced by dispersing 100 mg of Si-NPs in Preparation Example 1 in 25 ml of methanol, and irradiating the methanol with ultrasonic waves for 5 minutes.

### Example 2

Si-NPs surface-modified with an ethoxy group were produced by dispersing 100 mg of Si-NPs in Preparation Example 1 in 25 ml of ethanol, and irradiating the ethanol with ultrasonic waves for 5 minutes.

### Example 3

Si-NPs surface-modified with an isopropoxy group were produced by dispersing 100 mg of Si-NPs in Preparation Example 1 in 25 ml of isopropyl alcohol, and irradiating the isopropyl alcohol with ultrasonic waves for 5 minutes.

### Example 4

Si-NPs surface-modified with a 2-aminoalkoxy group were produced by dispersing 100 mg of Si-NPs in Preparation Example 1 in 25 ml of 2-aminoalcohol, and irradiating the 2-aminoalcohol with ultrasonic waves for 5 minutes.

### Example 5

Si-NPs surface-modified with a butylphenoxy group were produced by dispersing 100 mg of Si-NPs in Preparation Example 1 in a mixture consisting of 0.5 ml of butylphenol and 25 ml of dichloromethane, and irradiating the mixture with ultrasonic waves for 5 minutes.

### Example 6

Si-NPs surface-modified with an acetic acid group were produced by dispersing 100 mg of Si-NPs in Preparation Example 1 in a mixture consisting of 0.5 ml of acetic acid and 25 ml of dichloromethane, and irradiating the mixture with ultrasonic waves for 5 minutes.

### Example 7

Si-NPs surface-modified with a stearic acid group were produced by dispersing 100 mg of Si-NPs in Preparation Example 1 in a mixture consisting of 0.5 g of stearic acid and 25 ml of dichloromethane, and irradiating the mixture with ultrasonic waves for 5 minutes.

### Example 8

Si-NPs surface-modified with an acetylsalicylic acid group were produced by dispersing 100 mg of Si-NPs in Preparation Example 1 in a mixture consisting of 0.1 g of acetylsalicylic acid and 25 ml of dichloromethane, and irradiating the mixture with ultrasonic waves for 5 minutes.

### Example 9

Si-NPs surface-modified with a hydroxyl group of boric acid were produced by dispersing 100 mg of Si-NPs in Preparation Example 1 in 25 ml of an aqueous boric acid solution (boric acid and distilled water were mixed at a weight ratio of 5 : 95), and irradiating the aqueous boric acid solution with ultrasonic waves for 5 minutes.

### Example 10

Si-NPs surface-modified with a hydroxyl group of water were produced by dispersing 100 mg of Si-NPs in Preparation Example 1 in 25 ml of an aqueous organic solvent solution (water and methylene chloride were mixed at a weight ratio of 1 : 1), and irradiating the aqueous organic solvent solution with ultrasonic waves for 5 minutes.

### Example 11

SiB₄-NPs surface-modified with a methoxy group were produced by dispersing 100 mg of SiB₄-NPs in Preparation Example 2 in 25 ml of methanol, and irradiating the methanol with ultrasonic waves for 5 minutes.

### Example 12

SiB₄-NPs surface-modified with an ethoxy group were produced by dispersing 100 mg of SiB₄-NPs in Preparation Example 2 in 25 ml of ethanol, and irradiating the ethanol with ultrasonic waves for 5 minutes.

### Example 13

SiB₄-NPs surface-modified with an isopropoxy group were produced by dispersing 100 mg of SiB₄-NPs in Preparation Example 2 in 25 ml of isopropyl alcohol, and irradiating the isopropyl alcohol with ultrasonic waves for 5 minutes.

### Example 14

SiB₄-NPs surface-modified with a 2-aminoalkoxy group were produced by dispersing 100 mg of SiB₄-NPs in Preparation Example 2 in 25 ml of 2-aminoalcohol, and irradiating the 2-aminoalcohol with ultrasonic waves for 5 minutes.

### Example 15

SiB₄-NPs surface-modified with a butylphenoxy group were produced by dispersing 100 mg of SiB₄-NPs in Preparation Example 2 in a mixture consisting of 0.5 ml of butylphenol and 25 ml of dichloromethane, and irradiating the mixture with ultrasonic waves for 5 minutes.

### Example 16

SiB₄-NPs surface-modified with an acetic acid group were produced by dispersing 100 mg of SiB₄-NPs in Preparation Example 2 in a mixture consisting of 0.5 ml of acetic acid and 25 ml of dichloromethane, and irradiating the mixture with ultrasonic waves for 5 minutes.

### Example 17

SiB₄-NPs surface-modified with a stearic acid group were produced by dispersing 100 mg of SiB₄-NPs in Preparation Example 2 in a mixture consisting of 0.5 g of stearic acid and 25 ml of dichloromethane, and irradiating the mixture with ultrasonic waves for 5 minutes.

### Example 18

SiB₄-NPs surface-modified with an acetylsalicylic acid group were produced by dispersing 100 mg of SiB₄-NPs in Preparation Example 2 in a mixture consisting of 0.1 g of acetylsalicylic acid and 25 ml of dichloromethane, and irradiating the mixture with ultrasonic waves for 5 minutes.

### Example 19

SiB₄-NPs surface-modified with a hydroxyl group of boric acid were produced by dispersing 100 mg of SiB₄-NPs in Preparation Example 2 in 25 ml of an aqueous boric acid solution (boric acid and distilled water were mixed at a weight ratio of 5 : 95), and irradiating the aqueous boric acid solution with ultrasonic waves for 5 minutes.

### Example 20

SiB₄-NPs surface-modified with a hydroxyl group of water were produced by dispersing 100 mg of SiB₄-NPs in Preparation Example 2 in 25 ml of an aqueous organic solvent solution (water and methylene chloride were mixed at a weight ratio of 1 : 1), and irradiating the aqueous organic solvent solution with ultrasonic waves for 5 minutes.

### Example 21

SiC-NPs surface-modified with a methoxy group were produced by dispersing 100 mg of SiC-NPs in Preparation Example 3 in 25 ml of methanol, and irradiating the methanol with ultrasonic waves for 5 minutes.

### Example 22

SiC-NPs surface-modified with an ethoxy group were produced by dispersing 100 mg of SiC-NPs in Preparation Example 3 in 25 ml of ethanol, and irradiating the ethanol with ultrasonic waves for 5 minutes.

### Example 23

SiC-NPs surface-modified with an isopropoxy group were produced by dispersing 100 mg of SiC-NPs in Preparation Example 3 in 25 ml of isopropyl alcohol, and irradiating the isopropyl alcohol with ultrasonic waves for 5 minutes.

### Example 24

SiC-NPs surface-modified with a 2-aminoalkoxy group were produced by dispersing 100 mg of SiC-NPs in Preparation Example 3 in 25 ml of 2-aminoalcohol, and irradiating the 2-aminoalcohol with ultrasonic waves for 5 minutes.

### Example 25

SiC-NPs surface-modified with a butylphenoxy group were produced by dispersing 100 mg of SiC-NPs in Preparation Example 3 in a mixture consisting of 0.5 ml of butylphenol and 25 ml of dichloromethane, and irradiating the mixture with ultrasonic waves for 5 minutes.

### Example 26

SiC-NPs surface-modified with an acetic acid group were produced by dispersing 100 mg of SiC-NPs in Preparation Example 3 in a mixture consisting of 0.5 ml of acetic acid and 25 ml of dichloromethane, and irradiating the mixture with ultrasonic waves for 5 minutes.

### Example 27

SiC-NPs surface-modified with a stearic acid group were produced by dispersing 100 mg of SiC-NPs in Preparation Example 3 in a mixture consisting of 0.5 g of stearic acid and 25 ml of dichloromethane, and irradiating the mixture with ultrasonic waves for 5 minutes.

### Example 28

SiC-NPs surface-modified with an acetylsalicylic acid group were produced by dispersing 100 mg of SiC-NPs in Preparation Example 3 in a mixture consisting of 0.1 g of acetylsalicylic acid and 25 ml of dichloromethane, and irradiating the mixture with ultrasonic waves for 5 minutes.

### Example 29

SiC-NPs surface-modified with a hydroxyl group of boric acid were produced by dispersing 100 mg of SiC-NPs in Preparation Example 3 in 25 ml of an aqueous boric acid solution (boric acid and distilled water were mixed at a weight ratio of 5 : 95), and irradiating the aqueous boric acid solution with ultrasonic waves for 5 minutes.

### Example 30

SiC-NPs surface-modified with a hydroxyl group of water were produced by dispersing 100 mg of SiC-NPs in Preparation Example 3 in 25 ml of an aqueous organic solvent solution (water and methylene chloride were mixed at a weight ratio of 1 : 1), and irradiating the aqueous organic solvent solution with ultrasonic waves for 5 minutes.

### Example 31

SiGe-NPs surface-modified with a methoxy group were produced by dispersing 100 mg of SiGe-NPs in Preparation Example 4 in 25 ml of methanol, and irradiating the methanol with ultrasonic waves for 5 minutes.

### Example 32

SiGe-NPs surface-modified with an ethoxy group were produced by dispersing 100 mg of SiGe-NPs in Preparation Example 4 in 25 ml of ethanol, and irradiating the ethanol with ultrasonic waves for 5 minutes.

### Example 33

SiGe-NPs surface-modified with an isopropoxy group were produced by dispersing 100 mg of SiGe-NPs in Preparation Example 4 in 25 ml of isopropyl alcohol, and irradiating the isopropyl alcohol with ultrasonic waves for 5 minutes.

### Example 34

SiGe-NPs surface-modified with a 2-aminoalkoxy group were produced by dispersing 100 mg of SiGe-NPs in Preparation Example 4 in 25 ml of 2-aminoalcohol, and irradiating the 2-aminoalcohol with ultrasonic waves for 5 minutes.

### Example 35

SiGe-NPs surface-modified with a butylphenoxy group were produced by dispersing 100 mg of SiGe-NPs in Preparation Example 4 in a mixture consisting of 0.5 ml of butylphenol and 25 ml of dichloromethane, and irradiating the mixture with ultrasonic waves for 5 minutes.

### Example 36

SiGe-NPs surface-modified with an acetic acid group were produced by dispersing 100 mg of SiGe-NPs in Preparation Example 4 in a mixture consisting of 0.5 ml of acetic acid and 25 ml of dichloromethane, and irradiating the mixture with ultrasonic waves for 5 minutes.

### Example 37

SiGe-NPs surface-modified with a stearic acid group were produced by dispersing 100 mg of SiGe-NPs in Preparation Example 4 in a mixture consisting of 0.5 g of stearic acid and 25 ml of dichloromethane, and irradiating the mixture with ultrasonic waves for 5 minutes.

### Example 38

SiGe-NPs surface-modified with an acetylsalicylic acid group were produced by dispersing 100 mg of SiGe-NPs in Preparation Example 4 in a mixture consisting of 0.1 g of acetylsalicylic acid and 25 ml of dichloromethane, and irradiating the mixture with ultrasonic waves for 5 minutes.

### Example 39

SiGe-NPs surface-modified with a hydroxyl group of boric acid were produced by dispersing 100 mg of SiGe-NPs in Preparation Example 4 in 25 ml of an aqueous boric acid solution (boric acid and distilled water were mixed at a weight ratio of 5 : 95), and irradiating the aqueous boric acid solution with ultrasonic waves for 5 minutes.

### Example 40

SiGe-NPs surface-modified with a hydroxyl group of water were produced by dispersing 100 mg of SiGe-NPs in Preparation Example 4 in 25 ml of an aqueous organic solvent solution (water and methylene chloride were mixed at a weight ratio of 1 : 1), and irradiating the aqueous organic solvent solution with ultrasonic waves for 5 minutes.

### Example 41

SiGeB-NPs surface-modified with a methoxy group were produced by dispersing 100 mg of SiGeB-NPs in Preparation Example 5 in 25 ml of methanol, and irradiating the methanol with ultrasonic waves for 5 minutes.

### Example 42

SiGeB-NPs surface-modified with an ethoxy group were produced by dispersing 100 mg of SiGe-NPs in Preparation Example 5 in 25 ml of ethanol, and irradiating the ethanol with ultrasonic waves for 5 minutes.

### Example 43

SiGeB-NPs surface-modified with an isopropoxy group were produced by dispersing 100 mg of SiGeB-NPs in Preparation Example 5 in 25 ml of isopropyl alcohol, and irradiating the isopropyl alcohol with ultrasonic waves for 5 minutes.

### Example 44

SiGeB-NPs surface-modified with a 2-aminoalkoxy group were produced by dispersing 100 mg of SiGeB-NPs in Preparation Example 5 in 25 ml of 2-aminoalcohol, and irradiating the 2-aminoalcohol with ultrasonic waves for 5 minutes.

### Example 45

SiGeB-NPs surface-modified with a butylphenoxy group were produced by dispersing 100 mg of SiGeB-NPs in Preparation Example 5 in a mixture consisting of 0.5 ml of butylphenol and 25 ml of dichloromethane, and irradiating the mixture with ultrasonic waves for 5 minutes.

### Example 46

SiGeB-NPs surface-modified with an acetic acid group were produced by dispersing 100 mg of SiGeB-NPs in Preparation Example 5 in a mixture consisting of 0.5 ml of acetic acid and 25 ml of dichloromethane, and irradiating the mixture with ultrasonic waves for 5 minutes.

### Example 47

SiGeB-NPs surface-modified with a stearic acid group were produced by dispersing 100 mg of SiGeB-NPs in Preparation Example 5 in a mixture consisting of 0.5 g of stearic acid and 25 ml of dichloromethane, and irradiating the mixture with ultrasonic waves for 5 minutes.

### Example 48

SiGeB-NPs surface-modified with an acetylsalicylic acid group were produced by dispersing 100 mg of SiGeB-NPs in Preparation Example 5 in a mixture consisting of 0.1 g of acetylsalicylic acid and 25 ml of dichloromethane, and irradiating the mixture with ultrasonic waves for 5 minutes.

### Example 49

SiGeB-NPs surface-modified with a hydroxyl group of boric acid were produced by dispersing 100 mg of SiGeB-NPs in Preparation Example 5 in 25 ml of an aqueous boric acid solution (boric acid and distilled water were mixed at a weight ratio of 5 : 95), and irradiating the aqueous boric acid solution with ultrasonic waves for 5 minutes.

### Example 50

SiGeB-NPs surface-modified with a hydroxyl group of water were produced by dispersing 100 mg of SiGeB-NPs in Preparation Example 5 in 25 ml of an aqueous organic solvent solution (water and methylene chloride were mixed at a weight ratio of 1 : 1), and irradiating the aqueous organic solvent solution with ultrasonic waves for 5 minutes.

### Example 51

SiGeC-NPs surface-modified with a methoxy group were produced by dispersing 100 mg of SiGeC-NPs in Preparation Example 6 in 25 ml of methanol, and irradiating the methanol with ultrasonic waves for 5 minutes.

### Example 52

SiGeC-NPs surface-modified with an ethoxy group were produced by dispersing 100 mg of SiGeC-NPs in Preparation Example 6 in 25 ml of ethanol, and irradiating the ethanol with ultrasonic waves for 5 minutes.

### Example 53

SiGeC-NPs surface-modified with an isopropoxy group were produced by dispersing 100 mg of SiGeC-NPs in Preparation Example 6 in 25 ml of isopropyl alcohol, and irradiating the isopropyl alcohol with ultrasonic waves for 5 minutes.

### Example 54

SiGeC-NPs surface-modified with a 2-aminoalkoxy group were produced by dispersing 100 mg of SiGeC-NPs in Preparation Example 6 in 25 ml of 2-aminoalcohol, and irradiating the 2-aminoalcohol with ultrasonic waves for 5 minutes.

### Example 55

SiGeC-NPs surface-modified with a butylphenoxy group were produced by dispersing 100 mg of SiGeC-NPs in Preparation Example 6 in a mixture consisting of 0.5 ml of butylphenol and 25 ml of dichloromethane, and irradiating the mixture with ultrasonic waves for 5 minutes.

### Example 56

SiGeC-NPs surface-modified with an acetic acid group were produced by dispersing 100 mg of SiGeC-NPs in Preparation Example 6 in a mixture consisting of 0.5 ml of acetic acid and 25 ml of dichloromethane, and irradiating the mixture with ultrasonic waves for 5 minutes.

### Example 57

SiGeC-NPs surface-modified with a stearic acid group were produced by dispersing 100 mg of SiGeC-NPs in Preparation Example 6 in a mixture consisting of 0.5 g of stearic acid and 25 ml of dichloromethane, and irradiating the mixture with ultrasonic waves for 5 minutes.

### Example 58

SiGeC-NPs surface-modified with an acetylsalicylic acid group were produced by dispersing 100 mg of SiGeC-NPs in Preparation Example 6 in a mixture consisting of 0.1 g of acetylsalicylic acid and 25 ml of dichloromethane, and irradiating the mixture with ultrasonic waves for 5 minutes.

### Example 59

SiGeC-NPs surface-modified with a hydroxyl group of boric acid were produced by dispersing 100 mg of SiGeC-NPs in Preparation Example 6 in 25 ml of an aqueous boric acid solution (boric acid and distilled water were mixed at a weight ratio of 5 : 95), and irradiating the aqueous boric acid solution with ultrasonic waves for 5 minutes.

### Example 60

SiGeC-NPs surface-modified with a hydroxyl group of water were produced by dispersing 100 mg of SiGeC-NPs in Preparation Example 6 in 25 ml of an aqueous organic solvent solution (water and methylene chloride were mixed at a weight ratio of 1 : 1), and irradiating the aqueous organic solvent solution with ultrasonic waves for 5 minutes.

### Example 61

Ge-NPs surface-modified with a methoxy group were produced by dispersing 100 mg of Ge-NPs in Preparation Example 7 in 25 ml of methanol, and irradiating the methanol with ultrasonic waves for 5 minutes.

### Example 62

Ge-NPs surface-modified with an ethoxy group were produced by dispersing 100 mg of Ge-NPs in Preparation Example 7 in 25 ml of ethanol, and irradiating the ethanol with ultrasonic waves for 5 minutes.

### Example 63

Ge-NPs surface-modified with an isopropoxy group were produced by dispersing 100 mg of Ge-NPs in Preparation Example 7 in 25 ml of isopropyl alcohol, and irradiating the isopropyl alcohol with ultrasonic waves for 5 minutes.

### Example 64

Ge-NPs surface-modified with a 2-aminoalkoxy group were produced by dispersing 100 mg of Ge-NPs in Preparation Example 7 in 25 ml of 2-aminoalcohol, and irradiating the 2-aminoalcohol with ultrasonic waves for 5 minutes.

### Example 65

Ge-NPs surface-modified with a butylphenoxy group were produced by dispersing 100 mg of Ge-NPs in Preparation Example 7 in a mixture consisting of 0.5 ml of butylphenol and 25 ml of dichloromethane, and irradiating the mixture with ultrasonic waves for 5 minutes.

### Example 66

Ge-NPs surface-modified with an acetic acid group were produced by dispersing 100 mg of Ge-NPs in Preparation Example 7 in a mixture consisting of 0.5 ml of acetic acid and 25 ml of dichloromethane, and irradiating the mixture with ultrasonic waves for 5 minutes.

### Example 67

Ge-NPs surface-modified with a stearic acid group were produced by dispersing 100 mg of Ge-NPs in Preparation Example 7 in a mixture consisting of 0.5 g of stearic acid and 25 ml of dichloromethane, and irradiating the mixture with ultrasonic waves for 5 minutes.

### Example 68

Ge-NPs surface-modified with an acetylsalicylic acid group were produced by dispersing 100 mg of Ge-NPs in Preparation Example 7 in a mixture consisting of 0.1 g of acetylsalicylic acid and 25 ml of dichloromethane, and irradiating the mixture with ultrasonic waves for 5 minutes.

### Example 69

Ge-NPs surface-modified with a hydroxyl group of boric acid were produced by dispersing 100 mg of Ge-NPs in Preparation Example 7 in 25 ml of an aqueous boric acid solution (boric acid and distilled water were mixed at a weight ratio of 5 : 95), and irradiating the aqueous boric acid solution with ultrasonic waves for 5 minutes.

### Example 70

Ge-NPs surface-modified with a hydroxyl group of water were produced by dispersing 100 mg of Ge-NPs in Preparation Example 7 in 25 ml of an aqueous organic solvent solution (water and methylene chloride were mixed at a weight ratio of 1 : 1), and irradiating the aqueous organic solvent solution with ultrasonic waves for 5 minutes.

### Example 71

GeB₄-NPs surface-modified with a methoxy group were produced by dispersing 100 mg of GeB₄-NPs in Preparation Example 8 in 25 ml of methanol, and irradiating the methanol with ultrasonic waves for 5 minutes.

### Example 72

GeB₄-NPs surface-modified with an ethoxy group were produced by dispersing 100 mg of GeB₄-NPs in Preparation Example 8 in 25 ml of ethanol, and irradiating the ethanol with ultrasonic waves for 5 minutes.

### Example 73

GeB₄-NPs surface-modified with an isopropoxy group were produced by dispersing 100 mg of GeB₄-NPs in Preparation Example 8 in 25 ml of isopropyl alcohol, and irradiating the isopropyl alcohol with ultrasonic waves for 5 minutes.

### Example 74

GeB₄-NPs surface-modified with a 2-aminoalkoxy group were produced by dispersing 100 mg of GeB₄-NPs in Preparation Example 8 in 25 ml of 2-aminoalcohol, and irradiating the 2-aminoalcohol with ultrasonic waves for 5 minutes.

### Example 75

GeB₄-NPs surface-modified with a butylphenoxy group were produced by dispersing 100 mg of GeB₄-NPs in Preparation Example 8 in a mixture consisting of 0.5 ml of butylphenol and 25 ml of dichloromethane, and irradiating the mixture with ultrasonic waves for 5 minutes.

### Example 76

GeB₄-NPs surface-modified with an acetic acid group were produced by dispersing 100 mg of GeB₄-NPs in Preparation Example 8 in a mixture consisting of 0.5 ml of acetic acid and 25 ml of dichloromethane, and irradiating the mixture with ultrasonic waves for 5 minutes.

### Example 77

GeB₄-NPs surface-modified with a stearic acid group were produced by dispersing 100 mg of GeB₄-NPs in Preparation Example 8 in a mixture consisting of 0.5 g of stearic acid and 25 ml of dichloromethane, and irradiating the mixture with ultrasonic waves for 5 minutes.

### Example 78

GeB₄-NPs surface-modified with an acetylsalicylic acid group were produced by dispersing 100 mg of GeB₄-NPs in Preparation Example 8 in a mixture consisting of 0.1 g of acetylsalicylic acid and 25 ml of dichloromethane, and irradiating the mixture with ultrasonic waves for 5 minutes.

### Example 79

GeB₄-NPs surface-modified with a hydroxyl group of boric acid were produced by dispersing 100 mg of GeB₄-NPs in Preparation Example 8 in 25 ml of an aqueous boric acid solution (boric acid and distilled water were mixed at a weight ratio of 5 : 95), and irradiating the aqueous boric acid solution with ultrasonic waves for 5 minutes.

### Example 80

GeB₄-NPs surface-modified with a hydroxyl group of water were produced by dispersing 100 mg of GeB₄-NPs in Preparation Example 8 in 25 ml of an aqueous organic solvent solution (water and methylene chloride were mixed at a weight ratio of 1 : 1), and irradiating the aqueous organic solvent solution with ultrasonic waves for 5 minutes.

### Example 81

GeC-NPs surface-modified with a methoxy group were produced by dispersing 100 mg of GeC-NPs in Preparation Example 9 in 25 ml of methanol, and irradiating the methanol with ultrasonic waves for 5 minutes.

### Example 82

GeC-NPs surface-modified with an ethoxy group were produced by dispersing 100 mg of GeC-NPs in Preparation Example 9 in 25 ml of ethanol, and irradiating the ethanol with ultrasonic waves for 5 minutes.

### Example 83

GeC-NPs surface-modified with an isopropoxy group were produced by dispersing 100 mg of GeC-NPs in Preparation Example 9 in 25 ml of isopropyl alcohol, and irradiating the isopropyl alcohol with ultrasonic waves for 5 minutes.

### Example 84

GeC-NPs surface-modified with a 2-aminoalkoxy group were produced by dispersing 100 mg of GeC-NPs in Preparation Example 9 in 25 ml of 2-aminoalcohol, and irradiating the 2-aminoalcohol with ultrasonic waves for 5 minutes.

### Example 85

GeC-NPs surface-modified with a butylphenoxy group were produced by dispersing 100 mg of GeC-NPs in Preparation Example 9 in a mixture consisting of 0.5 ml of butylphenol and 25 ml of dichloromethane, and irradiating the mixture with ultrasonic waves for 5 minutes.

### Example 86

GeC-NPs surface-modified with an acetic acid group were produced by dispersing 100 mg of GeC-NPs in Preparation Example 9 in a mixture consisting of 0.5 ml of acetic acid and 25 ml of dichloromethane, and irradiating the mixture with ultrasonic waves for 5 minutes.

### Example 87

GeC-NPs surface-modified with a stearic acid group were produced by dispersing 100 mg of GeC-NPs in Preparation Example 9 in a mixture consisting of 0.5 g of stearic acid and 25 ml of dichloromethane, and irradiating the mixture with ultrasonic waves for 5 minutes.

### Example 88

GeC-NPs surface-modified with an acetylsalicylic acid group were produced by dispersing 100 mg of GeC-NPs in Preparation Example 9 in a mixture consisting of 0.1 g of acetylsalicylic acid and 25 ml of dichloromethane, and irradiating the mixture with ultrasonic waves for 5 minutes.

### Example 89

GeC-NPs surface-modified with a hydroxyl group of boric acid were produced by dispersing 100 mg of GeC-NPs in Preparation Example 9 in 25 ml of an aqueous boric acid solution (boric acid and distilled water were mixed at a weight ratio of 5 : 95), and irradiating the aqueous boric acid solution with ultrasonic waves for 5 minutes.

### Example 90

GeC-NPs surface-modified with a hydroxyl group of water were produced by dispersing 100 mg of GeC-NPs in Preparation Example 9 in 25 ml of an aqueous organic solvent solution (water and methylene chloride were mixed at a weight ratio of 1 : 1), and irradiating the aqueous organic solvent solution with ultrasonic waves for 5 minutes.

### Example 91

CB₄-NPs surface-modified with a methoxy group were produced by dispersing 100 mg of CB₄-NPs in Preparation Example 10 in 25 ml of methanol, and irradiating the methanol with ultrasonic waves for 5 minutes.

### Example 92

CB₄-NPs surface-modified with an ethoxy group were produced by dispersing 100 mg of CB₄-NPs in Preparation Example 10 in 25 ml of ethanol, and irradiating the ethanol with ultrasonic waves for 5 minutes.

### Example 93

CB₄-NPs surface-modified with an isopropoxy group were produced by dispersing 100 mg of CB₄-NPs in Preparation Example 10 in 25 ml of isopropyl alcohol, and irradiating the isopropyl alcohol with ultrasonic waves for 5 minutes.

### Example 94

CB₄-NPs surface-modified with a 2-aminoalkoxy group were produced by dispersing 100 mg of CB₄-NPs in Preparation Example 10 in 25 ml of 2-aminoalcohol, and irradiating the 2-aminoalcohol with ultrasonic waves for 5 minutes.

### Example 95

CB₄-NPs surface-modified with a butylphenoxy group were produced by dispersing 100 mg of CB₄-NPs in Preparation Example 10 in a mixture consisting of 0.5 ml of butylphenol and 25 ml of dichloromethane, and irradiating the mixture with ultrasonic waves for 5 minutes.

### Example 96

CB₄-NPs surface-modified with an acetic acid group were produced by dispersing 100 mg of CB₄-NPs in Preparation Example 10 in a mixture consisting of 0.5 ml of acetic acid and 25 ml of dichloromethane, and irradiating the mixture with ultrasonic waves for 5 minutes.

### Example 97

CB₄-NPs surface-modified with a stearic acid group were produced by dispersing 100 mg of CB₄-NPs in Preparation Example 10 in a mixture consisting of 0.5 g of stearic acid and 25 ml of dichloromethane, and irradiating the mixture with ultrasonic waves for 5 minutes.

### Example 98

CB₄-NPs surface-modified with an acetylsalicylic acid group were produced by dispersing 100 mg of CB₄-NPs in Preparation Example 10 in a mixture consisting of 0.1 g of acetylsalicylic acid and 25 ml of dichloromethane, and irradiating the mixture with ultrasonic waves for 5 minutes.

### Example 99

CB₄-NPs surface-modified with a hydroxyl group of boric acid were produced by dispersing 100 mg of CB₄-NPs in Preparation Example 10 in 25 ml of an aqueous boric acid solution (boric acid and distilled water were mixed at a weight ratio of 5 : 95), and irradiating the aqueous boric acid solution with ultrasonic waves for 5 minutes.

### Example 100

CB₄-NPs surface-modified with a hydroxyl group of water were produced by dispersing 100 mg of CB₄-NPs in Preparation Example 10 in 25 ml of an aqueous organic solvent solution (water and methylene chloride were mixed at a weight ratio of 1 : 1), and irradiating the aqueous organic solvent solution with ultrasonic waves for 5 minutes.

### Example 101

C@Si-NPs surface-modified with a methoxy group were produced by dispersing 100 mg of C@Si-NPs in Preparation Example 11 in 25 ml of methanol, and irradiating the methanol with ultrasonic waves for 5 minutes.

### Example 102

C@Si-NPs surface-modified with an ethoxy group were produced by dispersing 100 mg of C@Si-NPs in Preparation Example 11 in 25 ml of ethanol, and irradiating the ethanol with ultrasonic waves for 5 minutes.

### Example 103

C@Si-NPs surface-modified with a 2-aminoalkoxy group were produced by dispersing 100 mg of C@Si-NPs in Preparation Example 11 in 25 ml of 2-aminoalcohol, and irradiating the 2-aminoalcohol with ultrasonic waves for 5 minutes.

### Example 104

C@Si-NPs surface-modified with an acetic acid group were produced by dispersing 100 mg of C@Si-NPs in Preparation Example 11 in 25 ml of acetic acid, and irradiating the acetic acid with ultrasonic waves for 5 minutes.

### Example 105

C@Si-NPs surface-modified with a hydroxyl group of boric acid were produced by dispersing 100 mg of C@Si-NPs in Preparation Example 11 in 25 ml of an aqueous boric acid solution (boric acid and distilled water were mixed at a weight ratio of 5 : 95), and irradiating the aqueous boric acid solution with ultrasonic waves for 5 minutes.

### Example 106

C@SiB₄-NPs surface-modified with a methoxy group were produced by dispersing 100 mg of C@SiB₄-NPs in Preparation Example 12 in 25 ml of methanol, and irradiating the methanol with ultrasonic waves for 5 minutes.

### Example 107

C@SiB₄-NPs surface-modified with an ethoxy group were produced by dispersing 100 mg of C@SiB₄-NPs in Preparation Example 12 in 25 ml of ethanol, and irradiating the ethanol with ultrasonic waves for 5 minutes.

### Example 108

C@SiB₄-NPs surface-modified with a 2-aminoalkoxy group were produced by dispersing 100 mg of C@SiB₄-NPs in Preparation Example 12 in 25 ml of 2-aminoalcohol, and irradiating the 2-aminoalcohol with ultrasonic waves for 5 minutes.

### Example 109

C@SiB₄-NPs surface-modified with an acetic acid group were produced by dispersing 100 mg of C@SiB₄-NPs in Preparation Example 12 in 25 ml of acetic acid, and irradiating the acetic acid with ultrasonic waves for 5 minutes.

### Example 110

C@SiB₄-NPs surface-modified with a hydroxyl group of boric acid were produced by dispersing 100 mg of C@SiB₄-NPs in Preparation Example 12 in 25 ml of an aqueous boric acid solution (boric acid and distilled water were mixed at a weight ratio of 5 : 95), and irradiating the aqueous boric acid solution with ultrasonic waves for 5 minutes.

### Example 111

C@SiC-NPs surface-modified with a methoxy group were produced by dispersing 100 mg of C@SiC-NPs in Preparation Example 13 in 25 ml of methanol, and irradiating the methanol with ultrasonic waves for 5 minutes.

### Example 112

C@SiC-NPs surface-modified with an ethoxy group were produced by dispersing 100 mg of C@SiC-NPs in Preparation Example 13 in 25 ml of ethanol, and irradiating the ethanol with ultrasonic waves for 5 minutes.

### Example 113

C@SiC-NPs surface-modified with a 2-aminoalkoxy group were produced by dispersing 100 mg of C@SiC-NPs in Preparation Example 13 in 25 ml of 2-aminoalcohol, and irradiating the 2-aminoalcohol with ultrasonic waves for 5 minutes.

### Example 114

C@SiC-NPs surface-modified with an acetic acid group were produced by dispersing 100 mg of C@SiC-NPs in Preparation Example 13 in 25 ml of acetic acid, and irradiating the acetic acid with ultrasonic waves for 5 minutes.

### Example 115

C@SiC-NPs surface-modified with a hydroxyl group of boric acid were produced by dispersing 100 mg of C@SiC-NPs in Preparation Example 13 in 25 ml of an aqueous boric acid solution (boric acid and distilled water were mixed at a weight ratio of 5 : 95), and irradiating the aqueous boric acid solution with ultrasonic waves for 5 minutes.

### Example 116

C@SiGe-NPs surface-modified with a methoxy group were produced by dispersing 100 mg of C@SiGe-NPs in Preparation Example 14 in 25 ml of methanol, and irradiating the methanol with ultrasonic waves for 5 minutes.

### Example 117

C@SiGe-NPs surface-modified with an ethoxy group were produced by dispersing 100 mg of C@SiGe-NPs in Preparation Example 14 in 25 ml of ethanol, and irradiating the ethanol with ultrasonic waves for 5 minutes.

### Example 118

C@SiGe-NPs surface-modified with a 2-aminoalkoxy group were produced by dispersing 100 mg of C@SiGe-NPs in Preparation Example 14 in 25 ml of 2-aminoalcohol, and irradiating the 2-aminoalcohol with ultrasonic waves for 5 minutes.

### Example 119

C@SiGe-NPs surface-modified with an acetic acid group were produced by dispersing 100 mg of C@SiGe-NPs in Preparation Example 14 in 25 ml of acetic acid, and irradiating the acetic acid with ultrasonic waves for 5 minutes.

### Example 120

C@SiGe-NPs surface-modified with a hydroxyl group of boric acid were produced by dispersing 100 mg of C@SiGe-NPs in Preparation Example 14 in 25 ml of an aqueous boric acid solution (boric acid and distilled water were mixed at a weight ratio of 5 : 95), and irradiating the aqueous boric acid solution with ultrasonic waves for 5 minutes.

### Example 121

C@SiGeB-NPs surface-modified with a methoxy group were produced by dispersing 100 mg of C@SiGeB-NPs in Preparation Example 15 in 25 ml of methanol, and irradiating the methanol with ultrasonic waves for 5 minutes.

### Example 122

C@SiGeB-NPs surface-modified with an ethoxy group were produced by dispersing 100 mg of C@SiGeB-NPs in Preparation Example 15 in 25 ml of ethanol, and irradiating the ethanol with ultrasonic waves for 5 minutes.

### Example 123

C@SiGeB-NPs surface-modified with a 2-aminoalkoxy group were produced by dispersing 100 mg of C@SiGeB-NPs in Preparation Example 15 in 25 ml of 2-aminoalcohol, and irradiating the 2-aminoalcohol with ultrasonic waves for 5 minutes.

### Example 124

C@SiGeB-NPs surface-modified with an acetic acid group were produced by dispersing 100 mg of C@SiGeB-NPs in Preparation Example 15 in 25 ml of acetic acid, and irradiating the acetic acid with ultrasonic waves for 5 minutes.

### Example 125

C@SiGeB-NPs surface-modified with a hydroxyl group of boric acid were produced by dispersing 100 mg of C@SiGeB-NPs in Preparation Example 15 in 25 ml of an aqueous boric acid solution (boric acid and distilled water were mixed at a weight ratio of 5 : 95), and irradiating the aqueous boric acid solution with ultrasonic waves for 5 minutes.

### Example 126

C@SiGeC-NPs surface-modified with a methoxy group were produced by dispersing 100 mg of C@SiGeC-NPs in Preparation Example 16 in 25 ml of methanol, and irradiating the methanol with ultrasonic waves for 5 minutes.

### Example 127

C@SiGeC-NPs surface-modified with an ethoxy group were produced by dispersing 100 mg of C@SiGeC-NPs in Preparation Example 16 in 25 ml of ethanol, and irradiating the ethanol with ultrasonic waves for 5 minutes.

### Example 128

C@SiGeC-NPs surface-modified with a 2-aminoalkoxy group were produced by dispersing 100 mg of C@SiGeC-NPs in Preparation Example 16 in 25 ml of 2-aminoalcohol, and irradiating the 2-aminoalcohol with ultrasonic waves for 5 minutes.

### Example 129

C@SiGeC-NPs surface-modified with an acetic acid group were produced by dispersing 100 mg of C@SiGeC-NPs in Preparation Example 16 in 25 ml of acetic acid, and irradiating the acetic acid with ultrasonic waves for 5 minutes.

### Example 130

C@SiGeC-NPs surface-modified with a hydroxyl group of boric acid were produced by dispersing 100 mg of C@SiGeC-NPs in Preparation Example 16 in 25 ml of an aqueous boric acid solution (boric acid and distilled water were mixed at a weight ratio of 5 : 95), and irradiating the aqueous boric acid solution with ultrasonic waves for 5 minutes.

### Example 131

C@Ge-NPs surface-modified with a methoxy group were produced by dispersing 100 mg of C@Ge-NPs in Preparation Example 17 in 25 ml of methanol, and irradiating the methanol with ultrasonic waves for 5 minutes.

### Example 132

C@Ge-NPs surface-modified with an ethoxy group were produced by dispersing 100 mg of C@Ge-NPs in Preparation Example 17 in 25 ml of ethanol, and irradiating the ethanol with ultrasonic waves for 5 minutes.

### Example 133

C@Ge-NPs surface-modified with a 2-aminoalkoxy group were produced by dispersing 100 mg of C@Ge-NPs in Preparation Example 17 in 25 ml of 2-aminoalcohol, and irradiating the 2-aminoalcohol with ultrasonic waves for 5 minutes.

### Example 134

C@Ge-NPs surface-modified with an acetic acid group were produced by dispersing 100 mg of C@Ge-NPs in Preparation Example 17 in 25 ml of acetic acid, and irradiating the acetic acid with ultrasonic waves for 5 minutes.

### Example 135

C@Ge-NPs surface-modified with a hydroxyl group of boric acid were produced by dispersing 100 mg of C@Ge-NPs in Preparation Example 17 in 25 ml of an aqueous boric acid solution (boric acid and distilled water were mixed at a weight ratio of 5 : 95), and irradiating the aqueous boric acid solution with ultrasonic waves for 5 minutes.

### Example 136

C@GeB₄-NPs surface-modified with a methoxy group were produced by dispersing 100 mg of C@GeB₄-NPs in Preparation Example 18 in 25 ml of methanol, and irradiating the methanol with ultrasonic waves for 5 minutes.

### Example 137

C@GeB₄-NPs surface-modified with an ethoxy group were produced by dispersing 100 mg of C@GeB₄-NPs in Preparation Example 18 in 25 ml of ethanol, and irradiating the ethanol with ultrasonic waves for 5 minutes.

### Example 138

C@GeB₄-NPs surface-modified with a 2-aminoalkoxy group were produced by dispersing 100 mg of C@GeB₄-NPs in Preparation Example 18 in 25 ml of 2-aminoalcohol, and irradiating the 2-aminoalcohol with ultrasonic waves for 5 minutes.

### Example 139

C@GeB₄-NPs surface-modified with an acetic acid group were produced by dispersing 100 mg of C@GeB₄-NPs in Preparation Example 18 in 25 ml of acetic acid, and irradiating the acetic acid with ultrasonic waves for 5 minutes.

### Example 140

C@GeB₄-NPs surface-modified with a hydroxyl group of boric acid were produced by dispersing 100 mg of C@GeB₄-NPs in Preparation Example 18 in 25 ml of an aqueous boric acid solution (boric acid and distilled water were mixed at a weight ratio of 5 : 95), and irradiating the aqueous boric acid solution with ultrasonic waves for 5 minutes.

### Example 141

C@GeC-NPs surface-modified with a methoxy group were produced by dispersing 100 mg of C@GeC-NPs in Preparation Example 19 in 25 ml of methanol, and irradiating the methanol with ultrasonic waves for 5 minutes.

### Example 142

C@GeC-NPs surface-modified with an ethoxy group were produced by dispersing 100 mg of C@GeC-NPs in Preparation Example 19 in 25 ml of ethanol, and irradiating the ethanol with ultrasonic waves for 5 minutes.

### Example 143

C@GeC-NPs surface-modified with a 2-aminoalkoxy group were produced by dispersing 100 mg of C@GeC-NPs in Preparation Example 19 in 25 ml of 2-aminoalcohol, and irradiating the 2-aminoalcohol with ultrasonic waves for 5 minutes.

### Example 144

C@GeC-NPs surface-modified with an acetic acid group were produced by dispersing 100 mg of C@GeC-NPs in Preparation Example 19 in 25 ml of acetic acid, and irradiating the acetic acid with ultrasonic waves for 5 minutes.

### Example 145

C@GeC-NPs surface-modified with a hydroxyl group of boric acid were produced by dispersing 100 mg of C@GeC-NPs in Preparation Example 19 in 25 ml of an aqueous boric acid solution (boric acid and distilled water were mixed at a weight ratio of 5 : 95), and irradiating the aqueous boric acid solution with ultrasonic waves for 5 minutes.

### Example 146

C@CB₄-NPs surface-modified with a methoxy group were produced by dispersing 100 mg of C@CB₄-NPs in Preparation Example 20 in 25 ml of methanol, and irradiating the methanol with ultrasonic waves for 5 minutes.

### Example 147

C@CB₄-NPs surface-modified with an ethoxy group were produced by dispersing 100 mg of C@CB₄-NPs in Preparation Example 20 in 25 ml of ethanol, and irradiating the ethanol with ultrasonic waves for 5 minutes.

### Example 148

C@CB₄-NPs surface-modified with a 2-aminoalkoxy group were produced by dispersing 100 mg of C@CB₄-NPs in Preparation Example 20 in 25 ml of 2-aminoalcohol, and irradiating the 2-aminoalcohol with ultrasonic waves for 5 minutes.

### Example 149

C@CB₄-NPs surface-modified with an acetic acid group were produced by dispersing 100 mg of C@CB₄-NPs in Preparation Example 20 in 25 ml of acetic acid, and irradiating the acetic acid with ultrasonic waves for 5 minutes.

### Example 150

C@CB₄-NPs surface-modified with a hydroxyl group of boric acid were produced by dispersing 100 mg of C@GeB₄-NPs in Preparation Example 20 in 25 ml of an aqueous boric acid solution (boric acid and distilled water were mixed at a weight ratio of 5 : 95), and irradiating the aqueous boric acid solution with ultrasonic waves for 5 minutes.

### Experimental Example

An antimicrobial composition having a carbon-group non-oxide nanoparticle concentration of 2 mM/L was produced by diluting the carbon-group non-oxide nanoparticles produced in the Preparation Examples and the Examples with water. After the antimicrobial composition was spray-coated on a shoe insert and dried by hot air for 6 hours, the antimicrobial performance thereof against Bacteria A (*Staphylococcus aureus* ATCC 6538) and Bacteria B (*Klebsiella pneumoniae* ATCC 4352) was measured in accordance with a test method of KS K 0693:2011, which is an antimicrobial test, and is shown in the following Tables 1 to 4.

In Tables 1 to 4, Comparative Example 1 is an antimicrobial test result through silica (SiO₂, Aldrich) nanoparticles. Specifically, Comparative Example 1 is a result of carrying out an antimicrobial treatment after producing silica nanoparticles into a 10 ppm solution, spraying the solution onto a shoe insert, and drying the solution. Further, Comparative Example 2 is an antimicrobial test result through zinc oxide (ZnO, Aldrich) nanoparticles. Specifically, Comparative Example 2 is a result of carrying out an antimicrobial treatment after producing zinc oxide nanoparticles into a 10 ppm solution, spraying the solution onto a shoe insert, and drying the solution.

**<Table 1>**

| Classification | Density of bacteria A and bacteria B (No. of bacteria/mL, initial) | Density of bacteria A (No. of bacteria/mL, 18 hours later) | Bacteria reduction rate of bacteria A (%, 18 hours later) | Density of bacteria B (No. of bacteria/mL, 18 hours later) | Bacteria reduction rate of bacteria B (%, after 18 hours elapsed) |
|---|---|---|---|---|---|
| Preparation Example 1 | 2.0x10⁴ | <10 | 99.9 | <10 | 99.9 |
| Preparation Example 2 | 2.0x10⁴ | <10 | 99.9 | <10 | 99.9 |
| Preparation Example 3 | 2.0x10⁴ | <10 | 99.9 | <10 | 99.9 |
| Preparation Example 4 | 2.0x10⁴ | <10 | 99.9 | <10 | 99.9 |
| Preparation Example 5 | 2.0x10⁴ | <10 | 99.9 | <10 | 99.9 |
| Preparation Example 6 | 2.0x10⁴ | <10 | 99.9 | <10 | 99.9 |
| Preparation Example 7 | 2.0x10⁴ | <10 | 99.9 | <10 | 99.9 |
| Preparation Example 8 | 2.0x10⁴ | <10 | 99.9 | <10 | 99.9 |
| Preparation Example 9 | 2.0x10⁴ | <10 | 99.9 | <10 | 99.9 |
| Preparation Example 10 | 2.0x10⁴ | <10 | 99.9 | <10 | 99.9 |
| Preparation Example 11 | 2.0x10⁴ | <10 | 99.9 | <10 | 99.9 |
| Preparation Example 12 | 2.0x10⁴ | <10 | 99.9 | <10 | 99.9 |
| Preparation Example 13 | 2.0x10⁴ | <10 | 99.9 | <10 | 99.9 |
| Preparation Example 14 | 2.0x10⁴ | <10 | 99.9 | <10 | 99.9 |
| Preparation Example 15 | 2.0x10⁴ | <10 | 99.9 | <10 | 99.9 |
| Preparation Example 16 | 2.0x10⁴ | <10 | 99.9 | <10 | 99.9 |
| Preparation Example 17 | 2.0x10⁴ | <10 | 99.9 | <10 | 99.9 |
| Preparation Example 18 | 2.0x10⁴ | <10 | 99.9 | <10 | 99.9 |
| Preparation Example 19 | 2.0x10⁴ | <10 | 99.9 | <10 | 99.9 |
| Preparation Example 20 | 2.0x10⁴ | <10 | 99.9 | <10 | 99.9 |
| Comparative Example 1 | 2.0x10⁴ | 1.1x10⁶ | 60.3 | 5.8x10⁶ | 77.6 |
| Comparative Example 2 | 2.0x10⁴ | 5.1x10⁵ | 81.7 | 2.5x10⁶ | 90.3 |

**<Table 2>**

| Classification | Density of bacteria A and bacteria B (No. of bacteria/mL, initial) | Density of bacteria A (No. of bacteria/mL, 18 hours later) | Bacteria reduction rate of bacteria A (%, 18 hours later) | Density of bacteria B (No. of bacteria/mL, 18 hours later) | Bacteria reduction rate of bacteria B (%, after 18 hours elapsed) |
|---|---|---|---|---|---|
| Example 1 | 2.0x10⁴ | 1.8x10² | 99.1 | <10 | 99.9 |
| Example 2 | 2.0x10⁴ | <10 | 99.9 | <10 | 99.9 |
| Example 3 | 2.0x10⁴ | 9.4x10² | 95.3 | <10 | 99.9 |
| Example 4 | 2.0x10⁴ | <10 | 99.9 | <10 | 99.9 |
| Example 5 | 2.0x10⁴ | <10 | 99.9 | 5.8x10² | 97.1 |
| Example 6 | 2.0x10⁴ | <10 | 99.9 | 1.8x10² | 99.1 |
| Example 7 | 2.0x10⁴ | 4.0x10² | 98 | 4.2x10² | 97.9 |
| Example 8 | 2.0x10⁴ | <10 | 99.9 | <10 | 99.9 |
| Example 9 | 2.0x10⁴ | <10 | 99.9 | <10 | 99.9 |
| Example 10 | 2.0x10⁴ | <10 | 99.9 | <10 | 99.9 |
| Example 11 | 2.0x10⁴ | 1.1x10² | 99.5 | <10 | 99.9 |
| Example 12 | 2.0x10⁴ | <10 | 99.9 | <10 | 99.9 |
| Example 13 | 2.0x10⁴ | <10 | 99.9 | 2.8x10² | 98.6 |
| Example 14 | 2.0x10⁴ | <10 | 99.9 | <10 | 99.9 |
| Example 15 | 2.0x10⁴ | 1.0x10² | 99.5 | <10 | 99.9 |
| Example 16 | 2.0x10⁴ | <10 | 99.9 | <10 | 99.9 |
| Example 17 | 2.0x10⁴ | <10 | 99.9 | <10 | 99.9 |
| Example 18 | 2.0x10⁴ | <10 | 99.9 | <10 | 99.9 |
| Example 19 | 2.0x10⁴ | <10 | 99.9 | <10 | 99.9 |
| Example 20 | 2.0x10⁴ | <10 | 99.9 | <10 | 99.9 |
| Example 21 | 2.0x10⁴ | <10 | 99.9 | <10 | 99.9 |
| Example 22 | 2.0x10⁴ | <10 | 99.9 | <10 | 99.9 |
| Example 23 | 2.0x10⁴ | 1.1x10² | 99.5 | 8.4x10² | 95.8 |
| Example 24 | 2.0x10⁴ | <10 | 99.9 | <10 | 99.9 |
| Example 25 | 2.0x10⁴ | <10 | 99.9 | 2.2x10² | 98.9 |
| Example 26 | 2.0x10⁴ | <10 | 99.9 | <10 | 99.9 |
| Example 27 | 2.0x10⁴ | <10 | 99.9 | <10 | 99.9 |
| Example 28 | 2.0x10⁴ | <10 | 99.9 | 8.4x10² | 95.8 |
| Example 29 | 2.0x10⁴ | <10 | 99.9 | <10 | 99.9 |
| Example 30 | 2.0x10⁴ | <10 | 99.9 | <10 | 99.9 |
| Example 31 | 2.0x10⁴ | 2.6x10² | 98.7 | <10 | 99.9 |
| Example 32 | 2.0x10⁴ | <10 | 99.9 | <10 | 99.9 |
| Example 33 | 2.0x10⁴ | 4.2x10² | 97.9 | <10 | 99.9 |
| Example 34 | 2.0x10⁴ | <10 | 99.9 | <10 | 99.9 |
| Example 35 | 2.0x10⁴ | <10 | 99.9 | 2.0x10² | 99 |
| Example 36 | 2.0x10⁴ | 7.0x10² | 96.5 | <10 | 99.9 |
| Example 37 | 2.0x10⁴ | <10 | 99.9 | 4.5x10² | 97.8 |
| Example 38 | 2.0x10⁴ | <10 | 99.9 | 1.0x10² | 99.5 |
| Example 39 | 2.0x10⁴ | <10 | 99.9 | <10 | 99.9 |
| Example 40 | 2.0x10⁴ | <10 | 99.9 | <10 | 99.9 |
| Example 41 | 2.0x10⁴ | 3.5x10² | 98.3 | <10 | 99.9 |
| Example 42 | 2.0x10⁴ | <10 | 99.9 | <10 | 99.9 |
| Example 43 | 2.0x10⁴ | 2.1x10² | 99 | <10 | 99.9 |
| Example 44 | 2.0x10⁴ | <10 | 99.9 | <10 | 99.9 |
| Example 45 | 2.0x10⁴ | <10 | 99.9 | 6.3x10² | 96.9 |
| Example 46 | 2.0x10⁴ | <10 | 99.9 | <10 | 99.9 |
| Example 47 | 2.0x10⁴ | 3.2x10² | 98.4 | <10 | 99.9 |
| Example 48 | 2.0x10⁴ | <10 | 99.9 | <10 | 99.9 |
| Example 49 | 2.0x10⁴ | <10 | 99.9 | <10 | 99.9 |
| Example 50 | 2.0x10⁴ | <10 | 99.9 | <10 | 99.9 |
| Comparative Example 1 | 2.0x10⁴ | 1.1x10⁶ | 60.3 | 5.8x10⁶ | 77.6 |
| Comparative Example 2 | 2.0x10⁴ | 5.1x10⁵ | 81.7 | 2.5x10⁶ | 90.3 |

**<Table 3>**

| Classification | Density of bacteria A and bacteria B (No. of bacteria/mL, initial) | Density of bacteria A (No. of bacteria/mL, 18 hours later) | Bacteria reduction rate of bacteria A (%, 18 hours later) | Density of bacteria B (No. of bacteria/mL, 18 hours later) | Bacteria reduction rate of bacteria B (%, after 18 hours elapsed) |
|---|---|---|---|---|---|
| Example 51 | 2.0x10⁴ | 1.1x10² | 99.5 | <10 | 99.9 |
| Example 52 | 2.0x10⁴ | <10 | 99.9 | <10 | 99.9 |
| Example 53 | 2.0x10⁴ | <10 | 99.9 | 2.7x10² | 98.7 |
| Example 54 | 2.0x10⁴ | <10 | 99.9 | <10 | 99.9 |
| Example 55 | 2.0x10⁴ | <10 | 99.9 | <10 | 99.9 |
| Example 56 | 2.0x10⁴ | 1.1x10² | 99.5 | 8.4x10² | 95.8 |
| Example 57 | 2.0x10⁴ | <10 | 99.9 | <10 | 99.9 |
| Example 58 | 2.0x10⁴ | <10 | 99.9 | <10 | 99.9 |
| Example 59 | 2.0x10⁴ | <10 | 99.9 | <10 | 99.9 |
| Example 60 | 2.0x10⁴ | <10 | 99.9 | <10 | 99.9 |
| Example 61 | 2.0x10⁴ | <10 | 99.9 | <10 | 99.9 |
| Example 62 | 2.0x10⁴ | 7.6x10² | 96.2 | 5.4x10² | 97.3 |
| Example 63 | 2.0x10⁴ | 1.2x10² | 99.4 | 4.7x10² | 97.7 |
| Example 64 | 2.0x10⁴ | <10 | 99.9 | <10 | 99.9 |
| Example 65 | 2.0x10⁴ | <10 | 99.9 | 3.0x10² | 98.5 |
| Example 66 | 2.0x10⁴ | 5.2x10² | 97.4 | 3.2x10² | 98.4 |
| Example 67 | 2.0x10⁴ | <10 | 99.9 | 1.4x10² | 99.3 |
| Example 68 | 2.0x10⁴ | 2.0x10² | 99 | 4.7x10² | 97.7 |
| Example 69 | 2.0x10⁴ | <10 | 99.9 | <10 | 99.9 |
| Example 70 | 2.0x10⁴ | <10 | 99.9 | <10 | 99.9 |
| Example 71 | 2.0x10⁴ | 4.2x10² | 97.9 | <10 | 99.9 |
| Example 72 | 2.0x10⁴ | 2.5x10² | 98.8 | <10 | 99.9 |
| Example 73 | 2.0x10⁴ | 1.2x10² | 99.4 | <10 | 99.9 |
| Example 74 | 2.0x10⁴ | <10 | 99.9 | <10 | 99.9 |
| Example 75 | 2.0x10⁴ | <10 | 99.9 | 5.8x10² | 97.1 |
| Example 76 | 2.0x10⁴ | <10 | 99.9 | <10 | 99.9 |
| Example 77 | 2.0x10⁴ | <10 | 99.9 | 1.8x10² | 99.1 |
| Example 78 | 2.0x10⁴ | 1.1x10² | 99.5 | <10 | 99.9 |
| Example 79 | 2.0x10⁴ | <10 | 99.9 | <10 | 99.9 |
| Example 80 | 2.0x10⁴ | <10 | 99.9 | <10 | 99.9 |
| Example 81 | 2.0x10⁴ | 5.9x10² | 97.1 | <10 | 99.9 |
| Example 82 | 2.0x10⁴ | 3.3x10² | 98.4 | <10 | 99.9 |
| Example 83 | 2.0x10⁴ | 1.4x10² | 99.3 | <10 | 99.9 |
| Example 84 | 2.0x10⁴ | <10 | 99.9 | <10 | 99.9 |
| Example 85 | 2.0x10⁴ | 1.8x10² | 99.1 | <10 | 99.9 |
| Example 86 | 2.0x10⁴ | <10 | 99.9 | <10 | 99.9 |
| Example 87 | 2.0x10⁴ | <10 | 99.9 | 1.5x10² | 99.3 |
| Example 88 | 2.0x10⁴ | <10 | 99.9 | 4.6x10² | 97.7 |
| Example 89 | 2.0x10⁴ | <10 | 99.9 | <10 | 99.9 |
| Example 90 | 2.0x10⁴ | <10 | 99.9 | <10 | 99.9 |
| Example 91 | 2.0x10⁴ | 6.0x10² | 97 | 4.8x10² | 99.9 |
| Example 92 | 2.0x10⁴ | 4.2x10² | 97.9 | 3.1x10² | 99.9 |
| Example 93 | 2.0x10⁴ | 2.3x10² | 98.9 | 2.5x10² | 99.9 |
| Example 94 | 2.0x10⁴ | <10 | 99.9 | <10 | 99.9 |
| Example 95 | 2.0x10⁴ | 2.6x10² | 98.7 | <10 | 99.9 |
| Example 96 | 2.0x10⁴ | <10 | 99.9 | <10 | 99.9 |
| Example 97 | 2.0x10⁴ | 4.2x10² | 97.9 | <10 | 99.9 |
| Example 98 | 2.0x10⁴ | <10 | 99.9 | <10 | 99.9 |
| Example 99 | 2.0x10⁴ | <10 | 99.9 | <10 | 99.9 |
| Example 100 | 2.0x10⁴ | <10 | 99.9 | <10 | 99.9 |
| Comparative Example 1 | 2.0x10⁴ | 1.1x10⁶ | 60.3 | 5.8x10⁶ | 77.6 |
| Comparative Example 2 | 2.0x10⁴ | 5.1x10⁵ | 81.7 | 2.5x10⁶ | 90.3 |

**<Table 4>**

| Classification | Density of bacteria A and bacteria B (No. of bacteria/mL, initial) | Density of bacteria A (No. of bacteria/mL, 18 hours later) | Bacteria reduction rate of bacteria A (%, 18 hours later) | Density of bacteria B (No. of bacteria/mL, 18 hours later) | Bacteria reduction rate of bacteria B (%, after 18 hours elapsed) |
|---|---|---|---|---|---|
| Example 101 | 2.0x10⁴ | 3.1x10² | 98.5 | <10 | 99.9 |
| Example 102 | 2.0x10⁴ | 2.4x10² | 98.8 | <10 | 99.9 |
| Example 103 | 2.0x10⁴ | <10 | 99.9 | <10 | 99.9 |
| Example 104 | 2.0x10⁴ | <10 | 99.9 | <10 | 99.9 |
| Example 105 | 2.0x10⁴ | <10 | 99.9 | <10 | 99.9 |
| Example 106 | 2.0x10⁴ | 1.2x10² | 99.4 | <10 | 99.9 |
| Example 107 | 2.0x10⁴ | 1.3x10² | 99.4 | <10 | 99.9 |
| Example 108 | 2.0x10⁴ | <10 | 99.9 | <10 | 99.9 |
| Example 109 | 2.0x10⁴ | <10 | 99.9 | <10 | 99.9 |
| Example 110 | 2.0x10⁴ | <10 | 99.9 | <10 | 99.9 |
| Example 111 | 2.0x10⁴ | 2.6x10² | 98.7 | <10 | 99.9 |
| Example 112 | 2.0x10⁴ | 3.0x10² | 98.5 | <10 | 99.9 |
| Example 113 | 2.0x10⁴ | <10 | 99.9 | <10 | 99.9 |
| Example 114 | 2.0x10⁴ | <10 | 99.9 | <10 | 99.9 |
| Example 115 | 2.0x10⁴ | <10 | 99.9 | <10 | 99.9 |
| Example 116 | 2.0x10⁴ | 2.8x10² | 98.6 | <10 | 99.9 |
| Example 117 | 2.0x10⁴ | 2.9x10² | 98.6 | <10 | 99.9 |
| Example 118 | 2.0x10⁴ | <10 | 99.9 | <10 | 99.9 |
| Example 119 | 2.0x10⁴ | <10 | 99.9 | <10 | 99.9 |
| Example 120 | 2.0x10⁴ | <10 | 99.9 | <10 | 99.9 |
| Example 121 | 2.0x10⁴ | <10 | 99.9 | <10 | 99.9 |
| Example 122 | 2.0x10⁴ | <10 | 99.9 | <10 | 99.9 |
| Example 123 | 2.0x10⁴ | <10 | 99.9 | <10 | 99.9 |
| Example 124 | 2.0x10⁴ | <10 | 99.9 | <10 | 99.9 |
| Example 125 | 2.0x10⁴ | <10 | 99.9 | <10 | 99.9 |
| Example 126 | 2.0x10⁴ | 1.2x10² | 99.9 | <10 | 99.9 |
| Example 127 | 2.0x10⁴ | <10 | 99.9 | <10 | 99.9 |
| Example 128 | 2.0x10⁴ | <10 | 99.9 | <10 | 99.9 |
| Example 129 | 2.0x10⁴ | <10 | 99.9 | <10 | 99.9 |
| Example 130 | 2.0x10⁴ | <10 | 99.9 | <10 | 99.9 |
| Example 131 | 2.0x10⁴ | 4.4x10² | 97.8 | <10 | 99.9 |
| Example 132 | 2.0x10⁴ | 2.6x10² | 98.7 | <10 | 99.9 |
| Example 133 | 2.0x10⁴ | <10 | 99.9 | <10 | 99.9 |
| Example 134 | 2.0x10⁴ | <10 | 99.9 | <10 | 99.9 |
| Example 135 | 2.0x10⁴ | <10 | 99.9 | <10 | 99.9 |
| Example 136 | 2.0x10⁴ | 2.8x10² | 98.6 | <10 | 99.9 |
| Example 137 | 2.0x10⁴ | 3.3x10² | 98.4 | <10 | 99.9 |
| Example 138 | 2.0x10⁴ | <10 | 99.9 | <10 | 99.9 |
| Example 139 | 2.0x10⁴ | <10 | 99.9 | <10 | 99.9 |
| Example 140 | 2.0x10⁴ | <10 | 99.9 | <10 | 99.9 |
| Example 141 | 2.0x10⁴ | 3.5x10² | 98.3 | <10 | 99.9 |
| Example 142 | 2.0x10⁴ | 4.1x10² | 98 | <10 | 99.9 |
| Example 143 | 2.0x10⁴ | <10 | 99.9 | <10 | 99.9 |
| Example 144 | 2.0x10⁴ | <10 | 99.9 | <10 | 99.9 |
| Example 145 | 2.0x10⁴ | <10 | 99.9 | <10 | 99.9 |
| Example 146 | 2.0x10⁴ | 4.4x10² | 97.8 | 4.2x10² | 97.9 |
| Example 147 | 2.0x10⁴ | 5.2x10² | 97.4 | 5.6x10² | 97.2 |
| Example 148 | 2.0x10⁴ | <10 | 99.9 | <10 | 99.9 |
| Example 149 | 2.0x10⁴ | <10 | 99.9 | <10 | 99.9 |
| Example 150 | 2.0x10⁴ | <10 | 99.9 | <10 | 99.9 |
| Comparative Example 1 | 2.0x10⁴ | 1.1x10⁶ | 60.3 | 5.8x10⁶ | 77.6 |
| Comparative Example 2 | 2.0x10⁴ | 5.1x10⁵ | 81.7 | 2.5x10⁶ | 90.3 |

As can be seen through Tables 1 to 4, as a result of the antimicrobial experiments through the nanoparticles according to the Preparation Examples and the Examples of the present invention, it could be seen that antimicrobial effects ranging from a minimum of 95.8% to a maximum of 99.9% were shown, and these values are remarkably higher than those of the antimicrobial experimental results according to Comparative Examples 1 and 2.

The above-described description of the present invention is provided for illustrative purposes, and the person skilled in the art to which the present invention pertains will understand that the present invention can be easily modified into other specific forms without changing the technical spirit or essential features of the present invention. Therefore, it should be understood that the above-described Examples are illustrative only in all aspects and are not restrictive. For example, each constituent element which is described as a singular form may be implemented in a distributed form, and similarly, constituent elements which are described as being distributed may be implemented in a combined form.

The scope of the present invention is represented by the claims to be described below, and it should be interpreted that the meaning and scope of the claims and all the changes or modified forms derived from the equivalent concepts thereto fall within the scope of the present invention.

## Claims

1. An antimicrobial agent comprising carbon-group non-oxide nanoparticles having an average particle size of 5 to 400 nm.

2. The antimicrobial agent of claim 1, further comprising a first oxide layer formed on a surface of the carbon-group non-oxide nanoparticles.

3. The antimicrobial agent of claim 1, wherein the carbon-group non-oxide nanoparticles are one selected from the group consisting of Si, Ge, B, C, and Sn, or an alloy or compound of two or more thereof.

4. The antimicrobial agent of claim 1, further comprising a carbon layer formed on the surface of the carbon-group non-oxide nanoparticles.

5. The antimicrobial agent of claim 4, wherein the carbon layer has a thickness of 100 nm or less.

6. The antimicrobial agent of claim 2, wherein one or more functional groups selected from the group consisting of a carboxyl group, a hydroxyl group, and an alkoxy group are bonded to a surface of the first oxide layer.

7. The antimicrobial agent of claim 2, further comprising a second oxide layer consisting of a boron oxide formed on a surface of the first oxide layer.

8. A method for producing an antimicrobial agent, the method comprising the steps of:
(a) producing carbon-group non-oxide nanoparticles having a first oxide layer formed thereon;
(b1) producing a mixed solution by mixing the carbon-group non-oxide nanoparticles with one selected from the group consisting of an alcohol, a carboxylic acid, an aqueous boric acid solution, and water; and
(c1) applying ultrasonic waves to the mixed solution.

9. A method for producing an antimicrobial agent, the method comprising the steps of:
(a) producing carbon-group non-oxide nanoparticles having a first oxide layer formed thereon;
(b2) producing a first mixed solution by mixing the carbon-group non-oxide nanoparticles with a first solution comprising boric acid and an organic solvent;
(c2) obtaining carbon-group non-oxide nanoparticles having a second oxide layer consisting of a boron oxide formed on the first oxide layer by applying ultrasonic waves to the first mixed solution;
(d) producing a second mixed solution by mixing the carbon-group non-oxide nanoparticles with one selected from the group consisting of an alcohol, a carboxylic acid, an aqueous boric acid solution, and water; and
(e) applying ultrasonic waves to the second mixed solution.

10. The method of claim 8 or 9, wherein Step (a) is performed by irradiating a mixed gas comprising one or more raw material gases comprising a carbon-group element and a catalyst gas with a laser.

11. The method of claim 10, wherein the catalyst gas is sulfur hexafluoride.

12. The method of claim 8 or 9, further comprising the step of forming a carbon layer on a surface of the carbon-group non-oxide nanoparticles by irradiating a mixture comprising the carbon-group non-oxide nanoparticles and a carbon-based gas with a laser after Step (a).

13. The method of claim 9, wherein in Step (b2), the organic solvent is non-polar.
